(19) Europäisches Patentamt
European Patent Office
Office européen des brevets

(11) EP 1 707 200 A1

(12) EUROPEAN PATENT APPLICATION

(43) Date of publication:
**04.10.2006 Bulletin 2006/40**

(21) Application number: **06112007.7**

(22) Date of filing: **30.03.2006**

(51) Int Cl.:
*A61K 31/421* (2006.01) *A61K 31/426* (2006.01)
*A61P 17/00* (2006.01) *A61P 17/02* (2006.01)
*A61P 17/04* (2006.01) *A61P 17/06* (2006.01)
*A61P 17/08* (2006.01) *A61P 17/10* (2006.01)

(84) Designated Contracting States:
**AT BE BG CH CY CZ DE DK EE ES FI FR GB GR HU IE IS IT LI LT LU LV MC NL PL PT RO SE SI SK TR**
Designated Extension States:
**AL BA HR MK YU**

(30) Priority:
**30.03.2005 DK 200500437**
**27.06.2005 DK 200500948**
**27.06.2005 DK 200500949**
**27.06.2005 US 694774 P**
**28.06.2005 US 695040 P**
**30.03.2005 DK 200500438**

(71) Applicant: **Astion Development A/S**
**2100 Copenhagen O (DK)**

(72) Inventor: **Weidner, Morten Sloth**
**2830, Virum (DK)**

(74) Representative: **Plougmann & Vingtoft A/S**
**Sundkrogsgade 9,**
**P.O. Box 831**
**2100 Copenhagen Ø (DK)**

# (54) Dermatological compositions comprising oxaprozin or a closely related compound for the treatment of dermatological diseases

(57) The invention relates to dermatological compositions of Oxaprozin or a closely related compound suitable adapted for the treatment of a dermatological disease, where at least two of the enzymes selected from protein tyrosine kinase Syk, protein tyrosine kinase ZAP-70 and phosphodiesterase IV play a role in mediating the dermatological disease. The invention also encompasses dermatological compositions for the treatment of pruritus.

EP 1 707 200 A1

## Description

### FIELD OF THE INVENTION

The present invention relates to pharmacology and to the pharmaceutical formulation of dermatological compositions suitable for topical application of Oxaprozin or a closely related compound in the treatment of dermatological diseases, particularly eczema.

### BACKGROUND OF THE INVENTION

It has now been discovered that Oxaprozin, which hitherto has been recognised as a nonsteroidal anti-inflammatory drug (NSAID), effectively inhibits the enzymes protein tyrosine kinase Syk, protein tyrosine kinase ZAP-70 and phosphodiesterase IV (PDE-IV) in pharmacologically relevant doses and effectively reduces the symptoms and inflammation in an animal model of contact dermatitis.

It is generally acknowledged that the term "nonsteroidal anti-inflammatory drugs" is used to describe compounds with a molecular formula based on a substituted phenol or benzene ring and which pharmacologic actions principally are related to the inhibition of the enzyme cyclooxygenase-1 (Cox-1) and to some extent also to the inhibition of cyclooxygenase-2 (Cox-2) *(see* Greaves MW. Pharmacology and significance of nonsteroidal anti-inflammatory drugs in the treatment of skin diseases. J Am Acad Dermatol 1987 April;16(4):751-64). Whereas Cox-1 derived prostaglandins are not produced in skin, the cox-2 enzyme produces prostaglandins at sites of inflammation for which reason the goal of pharmacologic anti-inflammatory therapy in the past has been to inhibit Cox-2 derived prostaglandins.

However, as discovered by the present inventor, Oxaprozin has a quite different pharmaco-dynamic and safe profile in comparison to other known NSAIDs.

The enzymatic activities of each of the enzymes protein tyrosine kinase Syk, protein tyrosine kinase ZAP-70 and phosphodiesterase IV (PDE-IV) are crucial steps in the inflammatory process and operate at a much higher hierarchic levels than the cyclooxygenase pathway. Therefore, Oxaprozin interferes with multiple crucial pathways in the inflammatory cascade, which renders this drug much more promising as an anti-inflammatory candidate, in particular with respect to the treatment of inflammatory dermatological diseases, such as eczemas, than previously expected.

Protein tyrosine kinases Syk and ZAP-70 are a class of enzymes that catalyze the transfer of a phosphate group from ATP to a tyrosine residue located on a protein substrate. They mediate the earliest detectable signalling response of the inflammation process upon activation of mast cells, T cells and B cells leading to multiple cascades of pro-inflammatory reactions. Protein tyrosine kinase Syk is involved in the cellular responses to degranulation, lipid mediator synthesis and cytokine production in inflammatory cells and it is expected that protein tyrosine kinase Syk takes part in allergic or inflammatory reactions through controlling the functions of mast cell, basophils, and eosinophils. The protein tyrosine kinase ZAP-70 is required for T-cell development and T-cell antigen receptor function.

The PDE-IV enzymes belong to the family of phosphodiesterases (PDE's) which are responsible for the hydrolysis of intracellular cyclic adenosine and guanosine monophosphate (cAMP and cGMP, respectively). The type IV phosphodiesterase is a cAMP-specific enzyme localized in airway smooth muscle cells as well as in immune and inflammatory cells. The type IV enzyme is a key enzyme in the hydrolysis of cAMP in mast cells, basophils, eosinophils, monocytes and lymphocytes.

Furthermore, in addition to the enzyme systems recognised by the present inventor, Oxaprozin inhibits both anandamide hydrolase (a fatty acid amide hydrolase) in neurons and NF-kappaB activation in inflammatory cells. Oxaprozin also induces apoptosis of activated monocytes in a dose-dependent manner (Dallegri, Franco. A review of the emerging profile of the anti-inflammatory drug oxaprozin. (Expert Opin Pharmacother 2005 May; 6(5):777-85).

Obviously, the present inventor has recognised the very great pharmacological potential of Oxaprozin, at least with respect to treating dermatological diseases, in that this single compound is able to modify several of the crucial and principal pathways of the immunological response *in vivo,* which usually requires the administration of several compounds to obtain the same effect.

While Oxaprozin and NSAIDs have been used for a long time in the treatment of systemic inflammatory diseases, like osteoarthritis and rheumatoid arthritis, the utility of Oxaprozin in the treatment of inflammatory dermatological diseases, such as eczemas, have not been emphasized or demonstrated before.

Some NSAIDs have been suggested and developed for the treatment of specific dermatological diseases. To be mentioned is acetylsalicylic acid, indomethacin and parfenac (marketed as bufexamac®) that have been used in the treatment of dermatological diseases for a long time. Acetylsalicylic acid has been used in the treatment of pruritus in atopic eczema; indomethacin has been used in the treatment of sunburned skin; and parfenac has been used in the treatment of eczema, dermatitis and perianal pruritus (Greaves MW. Pharmacology and significance of nonsteroidal anti-inflammatory drugs in the treatment of skin diseases, J Am Acad Dermatol 1987 April;16(4): 751-64).

Salicylic acid has been used in the treatment of psoriasis *(*Going SM, Guyer BM, Jarvie DR, Hunter JA. Salicylic

acid gel for scalp psoriasis. Clin Exp Dermatol 1986 May;11 (3):260-2) and in the treatment of pruritus (Thomsen JS, Simonsen L, Benfeldt E, Jensen SB, Serup J. The effect of topically applied salicylic compounds on serotonin-induced scratching behaviour in hairless rats. Exp Dermatol 2002 August;11 (4):370-5).

Topically applied indomethacin has been shown to respond in an animal model of contact dermatitis (Lowe NJ, Virgadamo F, Stoughton RB. Anti-inflammatory properties of a prostaglandin antagonist, a corticosteroid and indomethacin in experimental contact dermatitis. BrJ Dermatol 1977 April;96(4):433-8*)* and topical treatment with flubiprofen reduces human skin inflammation *(*BlackAK, Hensby CN, Greaves MW. The effect of topical flurbiprofen on human skin inflammation [proceedings]. Br J Clin Pharmacol 1980 January; 9(1):125P).

However, some NSAIDs have failed to show any clear ability to treat the inflammation of certain skin diseases. For example, one study demonstrates that topically applied indomethacin has poor effect in relieving the erythema and oedema in moderate to severe inflammation following treatment with cryotherapy. In comparison, the topical application of the steroid, clobetasol propionate, proved to have effect *(*Humphreys F, Spiro J. The effects of topical indomethacin and clobetasol propionate on post-cryotherapy inflammation. BrJ Dermatol 1995 May;132(5):762-5*).* Green and Shuster demonstrate that topical 1% indomethacin had no effect on chronic stable plaque psoriasis in human studies *(*Green CA, Shuster S. Lack of effect of topical indomethacin on psoriasis. Br J Clin Pharmacol 1987 September;24(3):381-4*).*

Unfortunately, the topical use of various NSAIDs is associated with significant cutaneous side effects. For example, Bufexamac is marketed for the treatment of pruritus and contact allergy, but the compound itself is reported to cause contact allergy. Furthermore, it is reported that aspirin and indometacin may induce urticarial reactions, whereas piroxicam can lead to phototoxic or photo allergic dermatitis. Ketoprofen and Bufexamac are recognised as major contact allergens (Gebhardt M and Wollina U. Cutaneous side-effects of nonsteroidal anti-inflammatory drugs (NSAID) in Z Rheumatol 1995 November;54(6):405-12*).* Diclofenac is another NSAID that is associated with cutaneous side-effects when applied topically in that irritant-type contact dermatitis may develop *(*Rivers JK and McLean DI. An open study to assess the efficacy and safety of topical 3% diclofenac in a 2.5% hyaluronic acid gel for the treatment of actinic keratoses. Arch Dermatol 1997 October;133(10):1239-42).

Thus, the findings of the present inventor are quite surprising because unlike other used NSAIDs, Oxaprozin is found very effective and safe in treating dermatological diseases. In considering the present invention and the treatment of inflamed tissue of the skin, Oxaprozin should no longer be regarded as a typical NSAID, because its principal pharmacodynamic properties relevant to inflamed skin do not include cyclooxygenase (Cox-1/Cox-2) inhibition. In fact, Oxaprozin is one of the NSAIDs with poorest Cox-2 inhibitory activity and selectivity for Cox-2 inhibition *(*Kawai S. Cyclooxygenase selectivity and the risk of gastrointestinal complications of various non-steroidal anti-inflammatory drugs. A clinical consideration. In Inflamma. Res. Supplement 2 (1998) S102-S106*).* Thus, the present inventor believes that Oxaprozin does not belong to the group of Cox-2 inhibitors.

According to the new findings there is a need for safe and effective topical application of Oxaprozin in the managing of dermatological diseases. However, Oxaprozin itself has a poor solubility in water. Accordingly, there remains a need for providing Oxaprozin-containing dermatological compositions that exhibit sufficient penetration within the outermost layer of the skin (epidermis) so as to achieve sufficient delivery of Oxaprozin within the skin for optimal local treatment of a dermatological disease.

A number of patent applications refer to dermatological compositions of NSAIDs, but none of these citations have recognised the problem with topical administration of Oxaprozin or have provided any solution to that problem.

US2005014729A1 and WO05009342A2 (Pharmacia Corporation) describe in general terms topical compositions of cyclooxygenase-2 inhibitors. According to US2005014729A1 and WO05009342A2, the Cox-2 inhibitor may optionally be provided in the form of pharmaceutically acceptable salt. Although, the applications mention Oxaprozin as an example of a Cox-2 inhibitor, the applications fail to explicitly disclose and teach dermatological compositions comprising Oxaprozin. Furthermore, the present inventor is of the opinion that Oxaprozin is in fact not a Cox-2 inhibitor.

The main object of WO05027977A2 (AGIS INDUSTRIES (1983) LTD) relates to dermatological gel formulations of Diclofenac that is essentially free of hyaluronic acid having a molecular weight of between 150,000 and 750,000 Daltons for the purpose of treating actinic keratosis. It is suggested to apply other NSAIDs including Oxaprozin in the gel compositions.

Several other citations relate to dermatological compositions of NSAIDs, but they all fail to emphasise applying the NSAID in the salt form:

JP7316075A2 (POLA CHEM IND INC) relates to dermatological compositions (cream formulations) intended for the treatment of dry skin by topically administering an NSAID, preferably bufexamac, indomethacin, ibuprofen or tenoxicam, together with 10-20% of a water-retaining agent.

WO0059475A1 (Lipocine) relates to dermatological compositions comprising an ionisable NSAID and a carrier comprising a compound capable of ionising the NSAID (2-aminoethanol is suggested), a surfactant and a triglyceride.

WO02074290(AGIS INDUSTRIES (1983) LTD) relates to topically administrable compositions of NSAID's (specifically piroxicam, tenoxicam, tolmetin, diclofenac and ketorolac) in combination with a secondary therapeutic agent, such as an antibiotic, antibacterial, antiviral, anaesthetic, analgesic, antiallergic, corticosteroid, retinoid, antihistamine,

immunosuppressant and antiproliferative medications, and mixtures thereof.

US2005232957A1 (Katz K) relates to dermatological compositions for the treatment of dry skin by administering specific or non-specific cox inhibitors.

Ophthalmic formulations have also been suggested (WO9531968 and WO0205815).

Moreover, a number of patent applications relate to dermatological compositions of Oxaprozin in combination with other therapeutically active agents, such as opiates (WO9709973), salicylic acid (US 5804572), farnesol (WO0062743), antiviral agent (WO0069255 and WO04056349), xanthines (WO0069406), ferrulic acid, caffeic acid, or tannic acid (WO02069963), selective cox-2 inhibitor such as celecoxib (WO02096435), anti-viral agents (WO03059347), antibiotics (WO03061704), retinol (WO03105806), indole (EP1424325), EP-3 antagonists (EP1426059), capsaicin (WO04056305) and lactam (WO04093796).

Water-free dermatological compositions of Oxaprozin are suggested in WO9810742, while the invention of US20030157138 relates to dermatological compositions designed in a manner ensuring liquefying of the composition upon application to the skin by incorporating in the dermatological composition 1-25 percent of a solidifying agent and 75-99 percent of a hydrophobic solvent.

The present inventor has also found that a particular salt of Oxaprozin, namely the monoethanolamine salt, provides excellent properties when applied in a dermatological formulation. Notably, none of the above mentioned patents or patent applications emphasise the use of such a salt in dermatological compositions. Salts of Oxaprozin have earlier been described in generally terms in US2005014729A1 and WO05009342A2 (Pharmacia Corporation). The patent application WO 9744022 relates to potassium, sodium or Tris salts of Oxaprozin for use in orally administrable compositions and US 4,465,838 relates to Oxaprozin calcium salt for use in oral administration.

Monoethanolamine is a biological amine chemically designated as 2-aminoethanol (olamine). The amine is the natural base of certain phospholipids present in the mammalian cell membrane and is used for various industrial purposes.

Thus, the present invention provides dermatological compositions that are effective and safe in the treatment of dermatological diseases. This is quite surprising because many attempts have been made over time to apply NSAIDs in the treatment of dermatological diseases, but the success has been limited so far because of too low effect and significant skin irritation.

## SUMMARY OF THE INVENTION

Surprisingly, the present inventor has found that Oxaprozin, unlike other NSAIDs possess a strong therapeutic potential in the management of dermatological diseases, where the inhibition of at least one of the enzymes Protein tyrosine kinase Syk; Protein tyrosine kinase ZAP-70; and phosphodiesterase IV (PDE-IV) in the skin will lead to alleviation, suppression or removal of the inflammation of a dermatological disease. Such dermatological diseases are in particular thought to include various forms of eczemas, such as contact dermatitis, atopic dermatitis and hand eczema. Furthermore, such inhibition will also remove, alleviate or reduce the predominant symptom of these diseases, namely pruritus, and other symptoms such as erythema and oedema.

Experimental data shown herein clearly verify the significant immediate and complete alleviation of the characteristic symptoms of inflammation associated with various forms of eczemas (See examples 2 (irritant contact dermatitis and atopic dermatitis), 3 (insect bite inflammation), 4 (psoriasis) and 5 (oxazolone induced contact dermatitis in mice). The human data shown herein clearly demonstrates the significant immediate and complete alleviation of pruritus in patients suffering from contact dermatitis, atopic dermatitis and psoriasis. Furthermore, erythema and scaling of the skin were also improved during the first 1-2 weeks of treatment indicating a therapeutic effect on the underlying disease causing the pruritus. Oxaprozin displayed, at clinically relevant doses, a strong inhibiting effect at least comparable to that achieved with the strong steroid betamethasone-17-valerate.

The present inventor puts forward the hypothesis that the convincing effect observed with Oxaprozin is associated with the excellent pharmacological properties of Oxaprozin, namely the inhibition, in micro molar concentrations of one or more of the enzymes; Protein tyrosine kinases Syk, Protein tyrosine kinases ZAP-70 and PDE-IV enzyme. Such micro molar concentrations of Oxaprozin are expected to be present in skin cells following topical administration but pharmacologically active doses are also expected to be present following systemic administration.

Contrary to existing therapeutic agents used for treating inflammatory dermatological diseases, such as eczemas, the treatments according to the present invention have the advantage of not being likely to be associated with any serious side effects, as Oxaprozin has been shown to be safe and well tolerated by the organism in pharmacologically relevant doses. For example, Oxaprozin (in the form of its monoethanolamine salt) does not produce any cutaneous side-effects in the form of sensitization, phototoxic reaction, or acute dermal irritation. Furthermore, a dermatological formulation of this invention showed good cutaneous tolerance even when applied consecutively in a concentration of 5% for 28 days.

Unfortunately, the physico-chemical properties of Oxaprozin render this molecule difficult to formulate satisfactorily in dermatological compositions intended for local treatment of the skin. A high concentration must be achieved

on the surface of the skin to obtain sufficient amounts penetrating into the relevant layers of the skin including the epidermis and dermis. The primary reason for this difficulty derives from the poor solubility of Oxaprozin in both the water phase and the lipid phase of a dermatological composition. The problem with poor solubility may be solved by improving the solubility of Oxaprozin by adding various solubilisers, such as ethyl alcohol, isopropyl alcohol or diethylene glycol monoethyl ether to the dermatological composition. Unfortunately such solubilisers are skin irritants that may aggravate the condition and often add unwanted properties to the compositions itself, e.g. reduced tolerability of the composition.

Furthermore, the compound itself may cause tolerability problems to skin because of its low pH value.

The present inventor has surprisingly found that the problem with Oxaprozin and closely related compounds is solved, at least in part, by providing the Oxaprozin or the closely related compound in the form of a water soluble salt. Such compositions provide sufficient amounts of the Oxaprozin to the diseased skin because the composition can contain even high concentrations of Oxaprozin without producing saturated solutions of Oxaprozin and introducing the risk of crystal growth of Oxaprozin. Furthermore, salts are obtained that are physiologically compatible with respect to the desired pH properties in skin, because the salts have a pH value corresponding to the basic group of Oxaprozin. In addition, it is surprisingly found that although the dissociated form of Oxaprozin will have poor affinity for penetrating the upper layer of the skin, stratum corneum, because it is in ionised form, it is found that the dissociated form of Oxaprozin will revert back to the non-ionised parent form when applied to skin which normally has a low pH value. The parent form will have a better affinity for stratum corneum. Thus, sufficient dermal uptake of Oxaprozin is achieved even after topical application of dissociated Oxaprozin.

Accordingly, a first aspect of the invention relates to a dermatological composition comprising Oxaprozin or a closely related compound in the form of a water-soluble salt, such as a salt having a solubility in water at 25°C of a least 5mg/ml, such as at least 10mg/ml, such as at least 20, 25, 30, 50 mg/ml; and a vehicle comprising one or more dermatologically acceptable ingredient(s) or carrier(s).

Advantageous, such compositions are physical and chemical stable; they do not cause tolerability problems in skin, such as erythema and open sores, and they effectively treats inflammatory dermatological diseases, in particularly eczemas.

The present inventor has also recognised that 2-aminoethanol is a very interesting counter ion to Oxaprozin, because of its balanced hydrophilic and lipophilic nature that will have high affinity for the stratum corneum meaning that this particular compound is easily removed from the upper layer of the skin through diffusion of stratum corneum. On the contrary, more hydrophilic counter ions tend to accumulate on the surface of the skin because they cannot easily penetrate stratum corneum, which will result in poorer diffusion of Oxaprozin in cases where the counter ion is of hydrophilic nature. Such counter ions are thought to encompass $K^+$, $Na^+$, $Ca^{2+}$ and TRIS, which have been selected for oral fast releasing compositions. Thus, the inventor has provided a salt with good physicochemical properties and which has good the skin permeability.

Therefore, the present inventor has found that the monoethanolamine salt of Oxaprozin or closely related derivatives has excellent properties, at least with respect to topical application of Oxaprozin and with respect to tolerability.

Thus, a second aspect the invention relates to the 2-aminoethanol salt of Oxaprozin or a closely related compound, where the 2-aminoethanol cation is associated with an Oxaprozin anion.

In still further aspects, the invention relates to the use of the dermatological composition of the invention for the preparation of a medicament for the treatment of an inflammatory dermatological disease, in particularly eczemas.

In the various aspects of the invention, the treatment or prevention of an inflammatory dermatological disease encompasses:

- the treatment of eczemas;
- the treatment of hypersensitivity reactions in skin;
- the treatment of type-IV allergies in skin;
- the treatment of type-I allergies in skin;
- the treatment or prevention of contact dermatitis;
- the treatment or prevention of allergic contact dermatitis;
- the treatment or prevention of irritant contact dermatitis;
- the treatment of atopic dermatitis;

■ the treatment of hand eczema; or

■ the alleviation, removal or prevention of one or more of the following symptoms of the skin selected from the group consisting of pruritus; oedema; erythema; and scaling in a subject with inflammatory dermatological disease.

Still other aspects relate to the use of the dermatological composition of the invention for the preparation of a medicament for the treatment of pruritus in general or pruritus associated with or caused by a dermatological disease mentioned herein, or pruritus associated with or caused by systemic medications, pruritus associated with or caused by exposure to water or pruritus associated with or caused by a systemic disease.

## DETAILED DESCRIPTION OF THE INVENTION

The present inventor has recognised the need for dermatological formulations adapted for topical administration of Oxaprozin or a closely related compound, preferably to diseased skin, such as particularly the skin of a subject having eczema. Importantly, such compositions ought to deliver sufficiently high local concentration of dissolved Oxaprozin to the dermis without the need of adding solubilisers to the dermatological composition.

Importantly, the dermatological compositions of this invention shall be suitable for being applied to skin suffering from a dermatological disease, such as dermatological diseases associated with or caused by hypersensitivity reactions, type-I and type IV allergy reactions, e.g. eczemas.

Unlike previous dermatological compositions of Oxaprozin or other NSAIDs, the present inventor has emphasised the requirement for providing Oxaprozin in the form of a water-soluble salt. Furthermore, the inventor has emphasised the need of applying water-based dermatological compositions, which may assist in obtaining the desired effect. For example, the dermatological composition itself (i.e. the vehicle) can modify stratum corneum so as to achieve the desired penetration rate or sufficient delivery of Oxaprozin or the closely related compound to normal or diseased skin. Thus, the vehicle may alter the solubility and/or diffusivity of the Oxaprozin salt of the invention within the stratum corneum. In an optimal formulation, the delivery of Oxaprozin or a closely related compound will result in high local concentrations of Oxaprozin or the related compound in skin cells.

Therefore, the present invention provides in one aspect a dermatological formulation comprising i) Oxaprozin or a closely related compound in a form of a water-soluble salt and ii) a vehicle comprising one or more dermatologically acceptable ingredient(s) or carrier(s).

The dermatological composition is intended to be topically applied to skin for the local treatment of the upper surfaces of the skin in a subject in need thereof. Thus, ophthalmic preparations and transdermal plasters are not necessarily the scope of the present invention and may in some embodiments be regarded as excluded embodiments of the dermatological compositions mentioned herein.

The term "skin" is meant to include skin of the entire embody including the scalp, the forehead, the head, arms, legs, breast, perianal area and so forth. The term "skin" is also meant to include various layers of the skin, such as stratum corneum, epidermis and dermis.

The term "water-soluble" is meant to define Oxaprozin or a closely related compound modified in a manner resulting in much higher water-solubility than Oxaprozin, such as 5, 10, 20, 25, 40, 50, 75, 100, 200, 250, 400 and 500 times higher. The solubility of Oxaprozin in water at 25°C is about 1.7 mg/ml. Therefore, a water-soluble modification of Oxaprozin or a closely related compound is meant to denote a modification resulting in a solubility of the modified Oxaprozin in water at 25°C of at least 5, 10, 20, 25, 30 and 50 mg/ml, such as preferable 75, 100, 150, 200, 250 or even at least 300 mg/ml.

The term "vehicle" is meant to define a liquid, solid or semi-solid base made of dermatologically acceptable excipients or carriers, wherein the water-soluble form of Oxaprozin or the closely related compound is dissolved in or suspended in, preferably dissolved in.

The term "dermatologically acceptable," as used herein, means that the ingredient, carrier or vehicle so described are suitable for use in contact with human keratinous tissue, in particular with respect to application to eczematous skin without undue toxicity, incompatibility, instability, allergic response, and the like.

The phrase "local presence of the salt in skin" is meant to include topical administration of the salt to skin with the presumption that systemic uptake of the salt is limited or nil. Topical administration implies the intention that less than 50% by weight, such as less than 40%, 30%, 25%, 20%, 15%, 10% or 5% by weight of the topically administered salt may enter the blood stream or be recovered in urine and faeces. A method of local treatment of a site of skin inflammation or skin irritation in a subject comprises applying the composition to a skin surface of the subject, preferably at a locus overlying or adjacent to the site of inflammation and/or irritation, and leaving the composition in place for a time period effective to permit delivery of a local therapeutic amount of the active agent.

The term "diseased skin" is in the present context intended to mean skin suffering from a dermatological disease, such as particularly eczemas.

The inventor has found that it is a prerequisite requirement of dermatological compositions of the invention that Oxaprozin is applied in a water-soluble form in order to make it possible to achieve high dose levels in skin, at least when it is considered to also achieve good tolerability and low skin irritation.

Water-soluble modifications of Oxaprozin or a closely related compound may be obtained by several means, e.g.

- by micronising Oxaprozin or a closely related compound into particles in the micro range, e.g. where more than 80% of the particles has a particle diameter less than 30 μm or even lesser may be one means;
- by providing Oxaprozin or a closely related compound as a molecular dispersion, e.g. where Oxaprozin is dissolved in a suitable solvent and then added to silica with high surface area under the formation of dry powder;
- by formation of complexes, such as inclusion complexes with cyclodextrins; and
- by formation of water-soluble salts.

It has been found that water-soluble salts of Oxaprozin or a closely related compound may be applied by simple reaction of the free acid group of the Oxaprozin or the R chain of the closely related compound with suitable bases to form pharmaceutically acceptable salts, such as base addition salts. The type of salt would depend on the specific condition to be treated and the specific type of formulation. Therefore, a plethora of base addition salts may be applied.

Examples of inorganic base addition salts encompass Na, K, Ca, Mg, Cu, Zn and Mn salts. Typically, organic bases for use in the preparation of a base addition salt are primary, secondary or tertiary amines including alkylphenylamine, ammonia, 2-aminoethanol, aminopyrimidine, aminopyridine, arginine, benethamine, benzathine, betaine, caffeine, choline, deanol, diethanolamine, diethylamine, 2-diethylaminoethanol, 2-dimethylaminoethanol, ethylenediamine, N- ethylmorpholine, N-ethylpiperidine, glucamine, glucosamine, glycinol, hydrabamine, imidazol, isopropylamine, meglumine, methylglucamine, morpholine, piperazine, piperidine, procaine, purine, pyrrolidine, theobromine, thiamine, triethanolamine, triethylamine, trimethylamine, tripropylamine, tromethamine, spermidine, and the like. Furthermore, base addition salts may be derived from the reaction with natural amino acids such as with glycine, alanine, valine, leucine, isoleucine, norleucine, tyrosine, cystine, cysteine, methionine, proline, hydroxy proline, histidine, omithine, lysine, arginine, serine, threonine, and phenylalanine and with unnatural amino acids such as D-isomers or substituted amino acids; guanidine, substituted guanidine wherein the substituents are selected from nitro, amino, alkyl, alkenyl, alkynyl, ammonium or substituted ammonium salts and aluminum salts.

The following are non-limiting examples of relevant salts:

- metal salts wherein the cation typically may be selected from of $Li^+$, $Na^+$, $K^+$, $Ca^{++}$, $Mg^{++}$, $Ca^{++}$, $Cu^+$, $Zn^+$ and $Mn^+$;

- Amine salts formed by the reaction of the free acid of Oxaprozin or a closely related compound with primary, secondary or tertiary amines including alkylphenylamine, ammonia, 2-aminoethanol, aminopyrimidine, aminopyridine, arginine, benethamine, benzathine, betaine, caffeine, choline, deanol, diethanolamine, diethylamine, 2-diethylaminoethanol, 2-dimethylaminoethanol, ethylenediamine, N- ethylmorpholine, N-ethylpiperidine, glucamine, glucosamine, glycinol, hydrabamine, imidazol, isopropylamine, meglumine, methylglucamine, morpholine, piperazine, piperidine, procaine, purine, pyrrolidine, theobromine, thiamine, triethanolamine, triethylamine, trimethylamine, tripropylamine, tromethamine, spermidine, and the like;

- Amine salts formed by the reaction of the free acid of Oxaprozin or a closely related compound with natural amino acids such as with glycine, alanine, valine, leucine, isoleucine, norleucine, tyrosine, cystine, cysteine, methionine, proline, hydroxy proline, histidine, omithine, lysine, arginine, serine, threonine, and phenylalanine and with unnatural amino acids such as D-isomers or substituted amino acids; guanidine, substituted guanidine wherein the substituents are selected from nitro, amino, alkyl, alkenyl, alkynyl, ammonium or substituted ammonium salts and aluminum salts.

It should be understood that any base may be applied in the formation of the water-soluble salt as long as the solubility in water at 25°C is more than about 5, 10, 20, 25, 30, 40 or 50 mg/ml, such as preferably more than 75, 100, 150 and 200 mg/ml.

Moreover, to obtain a water-soluble salt having a pH in water in the range of 6 to 8, the water-soluble salt is preferably made from bases having a pKa in the range of 8 to 12, preferably from 8.5 to 11, more preferably from 8.5 to 10.5. That is to say that the water-soluble salt may be made from organic amines where the amino group has a pKa in the range from 8-12, preferably from 8.5-11, more preferably from 8.5 to 10.5, such as about in the range of 8.5 to 10.

In currently interesting embodiments of the invention the salt of Oxaprozin or a closely related compound is primarily made with less hydrophilic counter ions because they will easily penetrate skin and be removed from the upper surface of the skin thereby avoiding clogging of skin cells that otherwise may appear with more hydrophilic counter ions. Such less hydrophilic counter ions are thought to encompass mono-ethanolamine and other organic counter ions with log P values in the same order as mono-ethanolamine or with molecular weight in the same order. Such counter ions

are thought to encompass organic amines or amino acids, such as at least ethylenediamine, diethanolamine, choline, L-lysine and glucosamine. Mono-ethanolamine and 2-aminoethanol is interchangeable terms used herein. Counter ions that are less usable according to the present invention encompasses inorganic counter ions, like $Li^+$, $Na^+$, $K^+$, $Ca^{++}$, $Mg^{++}$, $Ca^{++}$, $Cu^+$, $Zn^+$ and $Mn^+$ and some hydrophilic organic amines, such as TRIS.

The log P value of monoethanolamine is about -1.3 and the molecular weight about 61.1 Dalton. Therefore, other counter ions selected from organic amines with log P value below 0, preferably below -0.5 can be used. Ideally, such organic amines have a log P value between 0 and - 10, preferably between 0 and -5, even more preferably between 0 and - 3, such as between 0 and -2.5.

Dermatological compositions may be physically, chemically and biologically stabile and may not prevent the salt of the invention, at least to a great extent, from being delivered from the vehicle into stratum corneum. One problem to encounter with the present dermatological invention is the content of salt (content of electrolytes) in that various emulsifiers are sensitive to the content of electrolytes resulting in demulsifying and separation of the lipophilic and hydrophilic phase of an emulsion.

Furthermore, it might be desirable to achieve dermatological compositions wherein the systemic uptake is limited, even after application to diseased skin. Therefore, the dermatological composition has to be designed accordingly.

There are numerous parameters to be taken into account when designing a dermatological composition; 1) the concentration of the water soluble salt, 2) the ratio between the hydrophilic and lipophilic phase if present, 3) viscosity, 4) content of permeation enhancers, 5) selection of oily phase constituents such as lipids, emulsifiers, co-emulsifiers and surfactants and 6) pH. Such parameters will all affect the penetration rate of the Oxaprozin salt through stratum corneum or affect the amount of the water-soluble salt which can be retained within epidermis and/or the upper dermis.

The phrase hydrophilic phase is meant to denote aqueous solution, water and something 'likes water', i.e. a something that typically is electrically polarized and capable of forming hydrogen bonds with water molecules, enabling it to dissolve more readily in water than in oil or other "non-polar" solvents. The hydrophilic phase comprises typically from 20% to 100% by weight of water including sterile water, more preferably from 50% to 100% of water, in particular from 80 to 100% of water. The hydrophilic phase may further comprise other liquid hydrophilic ingredients and ingredients, which dissolve or suspend in the aqueous phase, such as antioxidants, polar solvents, surfactants, emulsifiers, permeation enhancers, pH adjusters, preservatives, colours and flavours.

The lipophilic phase is meant to denote an oily/fatty phase containing one or more fatty and oily components as described below, optionally combined with emulsifiers, surfactants, emollients, antioxidants, preservatives, colours and flavours.

Generally speaking, dermatological compositions may be provided in several designs such as in the form of an emulsion including a microemulsion and a liposome formulation, gel, solution, liniment, ointment, foam, spray, aerosol, microsponge, patch or powder.

In considering applying a dermatological composition to diseased skin and to achieve satisfactorily delivery of a water-soluble salt through the stratum corneum into the dermis, the composition may be adapted to such conditions. Diseased skin, such as skin affected by a dermatological disease as defined herein, in particularly eczemas, often suffer from disrupted surface and with less intact stratum corneum. Therefore, dermatological compositions which are easy to apply on diseased skin, in particular onto greater parts of the skin, and which results in evenly spreading of the Oxaprozin salt throughout the diseased skin is desirable. Such compositions are thought to encompass emulsions, gels, solutions, sprays, foams and aerosols, preferable emulsions that are soft and easy to apply to diseased skin.

Dermatological compositions of the invention are preferably water-based. The content of the hydrophilic phase may be critical to the proper penetration profile, sufficient retention of Oxaprozin in the diseased skin cells, if desired. As used herein, a water-based dermatological composition is meant to contain more than 40% of a hydrophilic phase by weight of the vehicle. The vehicle contains preferably more than 45%, 47%, 50%, 55% or 60% and up to more than 75% such as about 80%, 85% or 90% of a hydrophilic phase. Therefore, ointments as such are not necessarily embodiments of the invention in that they tend to be water free. Therefore, in some embodiments ointments are excluded from the scope of the invention. That is to say that dermatological composition of the invention comprises no less than 5%, such as less than 10%, such as less than 15% by weight of water in the vehicle.

Interesting embodiments of the invention encompasses vehicles further comprising a fatty component or oily component, which may constitute up to between 20 and 70% of the vehicle, such as preferably between 30 and 50% by weight of the vehicle. That is to say, that gel compositions made exclusively of hydrophilic ingredients are excluded in such embodiments.

The concentration of the water-soluble salt of Oxaprozin or a closely related compound may be adapted according to the desired penetration rate. An over all minimum concentration may be required in order to achieve a sufficient therapeutic response, the minimum concentration being at least of about 0.005%, such as at least 0.01, 0.02, 0.04, 0.06, 0.08, 0.1, 0.2, 0.5, 0.7 or 1% by weight of the vehicle. The upper limit concentration may be about the concentration equal to the saturation concentration of the water soluble salt in the hydrophilic phase of the vehicle. Typically, the concentration of the salt may be up to 25% by weight of the vehicle. Typically, the concentration ranges between about

0.002% and 20% by weight of the vehicle, more preferably between 0.01% and 10% by weight and even more preferably between 0.1% and 5% by weight.

Typically, the dermatological composition should comprise a salt of Oxaprozin or a closely related compound in an amount of at least 0.5% by weight, more preferably of at least 1% by weight, even more preferably of at least 1.5% by weight, still more preferably of at least 2 % by weight, such as about 2.5% by weight, about 3% by weight, about 3.5% by weight, about 4% by weight, about 4.5% by weight, about 5% by weight, about 5.5% by weight, about 6% by weight or about 7% by weight.

Although Oxaprozin or a closely related compound is well tolerated in skin, it may be considered to apply amounts of the actives that secure safe treatment. Therefore, the dermatological composition should comprise a salt of Oxaprozin or a closely related compound in an amount less than 7% by weight, more preferably less than 6.5 % by weight, even more preferably less than 6% by weight, still more preferably less than 5.5 % by weight, even still more preferably less than 5% by weight, such as less than 4.5% by weight, such as less than 4% by weight, such as less than 3.5% by weight.

Accordingly, preferred embodiments of the invention should comprise a salt of Oxaprozin or a closely related compound in an amount ranging between 0.5 and 10% by weight, such as between 0.5 and 8% by weight, preferably between 0.5 and 7% by weight, such as between 0.5 and 6% by weight, 0.5 and 5.5 % by weight, 0.5 and 5% by weight, 0.5 and 4.5% by weight, 0.5 and 4% by weight, 0.5 and 3.5% by weight, such as 0.5 and 3% by weight. Still more preferable embodiments of the invention, comprises a salt of Oxaprozin or a closely related compound or a salt thereof in an amount ranging between 1 and 7% by weight, preferably between 1 and 6.5% by weight, such as between 1 and 6% by weight, 1 and 5.5 % by weight, 1 and 5% by weight, 1 and 4.5% by weight, 1 and 4% by weight, 1 and 3.5% by weight, such as 1 and 3% by weight. Still more preferably embodiments of the invention, comprises a salt of Oxaprozin or a closely related compound in an amount ranging between 1.5 and 7% by weight, preferably between 1.5 and 6.5% by weight, such as between 1.5 and 6% by weight, 1.5 and 5.5 % by weight, 1.5 and 5% by weight, 1.5 and 4.5% by weight, 1.5 and 4% by weight, 1.5 and 3.5% by weight, such as 1.5 and 3% by weight.

In currently interesting embodiments of the invention, the dermatological composition comprises Oxaprozin or a closely related compound in an amount of about 1%, of about 1.5%, of about 2%, of about 2.5%, of about 3%, of about 3.5%, of about 4% by weight, of about 4.5% weight, of about 5% by weight or of about 6% by weight, preferably 2.5%; 3%, 3.5% or 4% by weight.

The required concentration of the salt in the vehicle may also be expressed as a function of the salts saturation solubility in the hydrophilic phase. The saturation solubility is meant to denote the maximum concentration of the salt that can be dissolved in the hydrophilic phase. Thus, the concentration of the salt in the hydrophilic phase may be 0.01% up to 100% of the saturation solubility, preferably about 0.1% up to 20% of the saturation solubility.

If desired, the pH of the hydrophilic phase may be adjusted by adding acceptable acids or bases such as diethanolamine, citric acid, ascorbic acid, lactic acid, triethanolamine, sodium hydroxide, hydrochloric acid and sodium phosphate. The pH of the hydrophilic phase of the dermatological composition may be in the range between 3 and 8, but preferably in the range from pH 5 and 8, even more preferably about 6.5 and 7.8.

In a currently interesting embodiment of the invention, the hydrophilic phase has a pH value between 6.8 and 7.5, such as between 7.0 and 7.5, such as about 7.2, 7.3 or 7.4. Such pH values are found to constitute an improved embodiment of the invention, in that lower pH values are undesirable because of risk of precipitation of the Oxaprozin salt and higher pH values are undesirable because of skin irritation.

Generally, the dermatological compositions of this invention may be in one of the following forms:

A water-in-oil emulsion: The Oxaprozin salt may be incorporated into an emulsion that includes a continuous phase of a hydrophobic phase and an aqueous phase that includes water and optionally one or more polar hydrophilic carrier(s) and salts. These emulsions may include oil-soluble or oil-swellable polymers as well as one or more emulsifier (s) that help to stabilize the emulsion. The Oxaprozin salt is preferably added to the aqueous phase.

An oil-in-water emulsion: The Oxaprozin salt may be emulsified into an emulsion comprising a discrete phase of a hydrophobic phase and a continuous aqueous phase that includes water and optionally one or more polar hydrophilic carrier(s) as well as salts, surfactants, emulsifiers, and other components. These emulsions may include water-soluble or water-swellable polymers as well as one or more emulsifier(s) that help to stabilize the emulsion.

A hydrophobic ointment: The Oxaprozin salt can be formulated into a hydrophobic base (e.g. by incorporating Oxaprozin in petrolatum, thickened or gelled water, insoluble oils, triglycerides, transcutol and the like) and optionally with a minor amount of a water soluble phase in which Oxaprozin is kept soluble.

Thickened Aqueous gels: The Oxaprozin salt can be formulated into an aqueous phase which has been thickened to achieve a high viscosity. The thickening can be furnished by suitable natural, modified natural or synthetic polymers as described below. Alternatively, the thickened aqueous gels can be thickened using suitable polyethoxylated alkyl chain surfactants that effectively thicken the composition as well as other non-ionic, cationic, or anionic emulsifier systems. Preferably, non-ionic emulsifier systems are chosen since ionic emulsifiers tend to be sensitive to the salt content. Examples on non-ionic systems are Polawax, Cosmowax, and Crothix emulsifying systems.

Hydrophilic gels: The Oxaprozin salt can be formulated into a continuous phase that includes at least one water soluble hydrophilic component other than water. The formulations may contain water up to about between 70 and 99% by weight, such as between 80 and 95% by weight. Lower levels may be suitable in some compositions. Suitable hydrophilic components include glycols such as glycerin, propylene glycol, butylene glycols, etc., polyethylene glycols (PEG), random or block copolymers of ethylene oxide, propylene oxide, and/or butylene oxide, polyalkoxylated surfactants having one or more hydrophobic moieties per molecule, silicone copolyols, as well as combinations thereof, and the like.

Foams: The Oxaprozin salt can be formulated as foam that typically include as the vehicle water, a lipophilic solvent, a surface-active agent, an emulsifier and a specific gelling agent. Such carriers, when placed in an aerosol container and combined with a liquefied gas propellant, create a non-translucent oil-in-water emulsion that is stable in its pre-dispensed state. Liquefied gas propellant is added to the carrier in an amount of about 3-18% by weight of the total composition. Upon release from the aerosol container, the carriers form breakable foam products, which are suitable for topical administration.

The various constituents that can be used in the dermatological compositions are typically:

- Oily components, which are constituents of the hydrophobic phase of the various dermatological compositions forms and which may be made of one of the following dermatologically acceptable ingredients or a mixture of two or more thereof: almond oil, castor oil, cacao butter, coconut oil, corn oil, cottonseed oil, linseed oil, olive oil, palm oil, peanut oil, poppy seed oil, rapeseed oil, sesame oil, soybean oil, sunflower oil, and teaseed oil), mineral oils, fatty oils, liquid paraffin, mineral oil, isopropyl myristate, beewax, cottonseed oil, cetosteraryl alcohol, lanolin, white soft paraffin, yellow soft paraffin, canola oil, cetyl alcohol (cetanol), peanut oil, oleic acid, isopropyl palmitate, castor oil, stearyl alcohol, jojoba oil, stearic acid and silicone oils.

- Fatty components, which are constituents of the hydrophobic phase of the various dermatological compositions forms and may be used in combination with or instead of the oil phase and typically includes one or more ingredients selected from beeswax, paraffin, petrolatum, triglycerides, cetyl palmitate, vegetable oils, sorbitan esters of fatty acids (Span), solid macrogols (polyethylene glycols), and condensation products between sorbitan esters of fatty acids and ethylene oxide, e.g. polyoxyethylene sorbitan monooleate (Tween). Typical fatty components may be selected from the group comprising petrolatum, paraffins, vegetable oils, animal fats, synthetic glycerides, waxes, lanolin, and liquid polyalkylsiloxanes. Typical fatty components are but not limited to solid macrogols (polyethylene glycols).

- Aqueous phase, which constitutes the hydrophilic phase and which mainly comprise water, hydrophilic solvents, surfactants, emulsifier, preservatives, pH adjusters, flavours, colours and other hydrophilic ingredients.

- Hydrophilic solvents which may be added to the aqueous phase, such as polar solvents in the form of water, propylene glycol, glycerol, sorbitol, ethanol, industrial methylated spirit, polyethylene glycols, propylene glycols, propylene carbonate, and triacetin.

- Lipophilic solvents which may be added to the lipophilic phase, such as non polar solvents in the form of isopropyl alcohol and medium chain triglycerides (MCT).

- Emollients, such as fatty acid mono, di or tri glycerides, and fatty acid esters, dodecane, squalane, cholesterol, isohexadecane, isononyl isononanoate, PPG Ethers, petrolatum, lanolin, safflower oil, castor oil, coconut oil, cottonseed oil, palm kernel oil, palm oil, peanut oil, soybean oil, polyol carboxylic acid esters, derivatives thereof and mixtures thereof.

- Emulsifiers (emulsifying agents), which may be added either to the aqueous phase or to the oil phase: Compositions of the present invention can include one or more emulsifiers to emulsify the composition. As used herein the term "emulsifier" means an amphiphilic molecule possessing both polar and non-polar regions which are covalently bound and capable of reducing the surface tension of water and for the interfacial tension between water and an immiscible liquid. The term is meant to include soaps, detergents, emulsifiers, surface active agents, and the like. The emulsifier can be cationic, anionic, non-ionic, or amphoteric. This includes a wide variety of conventional emulsifiers;

Non-ionic Emulsifiers. Exemplary non-ionic emulsifiers include, but are not limited to, Polyol esters including glycols (e.g. ethylene glycol, diethylene glycol, glycol stearate and propylene glycol monoesters of fatty acids (propylene glycol stearate, propylene glycol oleate or propylene glycol palmitostearate)) and glycerol esters (e.g. glyceryl stearate, glyceryl monooleate, glycerylmonolaurate, glyceryl ricinolate, glyceryl monocaprylate);

Sorbitan derivatives, that consists of esters of cyclic anhydrides of sorbitol with a fatty acid (C12-C18). Sorbitan derivatives are divided into two groups i) sorbitan esters of fatty acids (e.g. sorbitan monolaurate, sorbitan monooleate, sorbitan monostearate (SPAN 60™), sorbitan monopalmitate, sorbitan sesquioleate, sorbitan trioleate or sorbitan tristearate) and ii) polyoxyethylene sorbitan esters (e.g. polyoxyethylene sorbitan monostearate (TWEEN 60 ™), polyoxyethylene sorbitan tristearate (TWEEN 65™), polyoxyethlene sorbitan monooleate (TWEEN 80™);
Polyoxyethylene esters (also called macrogol esters) are mixtures of mono- or di-fatty acids esters (from C12 to C18) of polyoxyethylene glycol (PEG), e.g. stearate esters of PEG (PEG-40, PEG-50 and PEG-55), laurate, oleate, and myristate esters of PEG;
Polyoxyethylene ethers are ethers of macrogol and fatty alcohols, such as ethers of the alcohols: stearyl (steareth emulsifiers), cetosteraryl (ceteareth emulsifiers) and oleyl (oleth emulsifiers);
Poloxamers that are polyoxyethylene-polyoxypropylene derivatives with polyoxyethylene groups (e.g. poloxamers-188);
Nonylphenyl ethers (nonoxinols) that are ethoxylated nonylphenols;
Propylene glycol Diacetate;
Polyvinyl alcohol;
Alkanolamides prepared from reaction of fatty acids with mono or diethanolamine;
Fatty alcohols (e.g. cetyl alcohol and stearate alcohol); alkyl glucosides; alkyl polyglucosides; polyhydroxy fatty acid amides; sucrose esters; fatty acid alkanolamides; ethoxylated fatty acids; ethoxylated aliphatic acids; ethoxylated fatty alcohols (e.g., octyl phenoxy polyethoxyethanoal available under the trade name TRITON X-100 and nonyl phenoxy poly(ethyleneoxy) ethanol available under the trade name NONIDET P-40, both from Sigma, St. Louis, MO); ethoxylated and/or propoxylated aliphatic alcohols; ethoxylated glycerides; ethoxylated propoxylated block copolymers such as PLURONIC and TETRONIC surfactants available from BASF.

Cationic Emulsifiers. Exemplary cationic emulsifiers include, but are not limited to: salts of primary, secondary, or tertiary fatty amines that optionally may be polyoxyalkylenated; quaternary ammonium salts, such as tetraalkylammonium, alkylamidoalkyltrialkylammonium, trialkylbenzylammonium, trialkylhydroxyalkylammonium, or alkylpyridinium halides (preferably chlorides or bromides) as well as other anionic counter-ions, such as but not limited to, alkyl sulfates, such as but not limited to, methosulfate and ethosulfate; imidazoline derivatives; amine oxides of a cationic nature (e.g., at an acidic pH). Examples of amineoxide emulsifiers include those which are lauryldimethylamine oxide, laurylamidopropyldimethylamine oxide, and cetyl amine oxide.

Anionic Emulsifiers. Exemplary anionic emulsifiers include, but are not limited to, sarcosinates, glutamates, alkyl sulfates, sodium or potassium alkyleth sulfates, ammonium alkyleth sulfates, ammonium laureth-n-sulfates, laweth-n-sulfates, isethionates, glycerylether sulfonates, sulfosuccinates, alkylglyceryl ether sulfonates, alkyl phosphates, aralkyl phosphates, alkylphosphonates, and aralkylphosphonates. These anionic emulsifiers may have a metal or organic ammonium counterion.

Amphoteric Emulsifiers. Emulsifiers of the amphoteric type include emulsifiers having tertiary amine groups, which may be protonated, as well as quaternary amine containing zwitterionic emulsifiers. Examples of such amphoteric emulsifiers include, but are not limited to: certain betaines such as cocobetaine and cocamidopropyl betaine; monoacetates such as sodium lauroamphoacetate; diacetates such as disodium lauroamphoacetate; amino- and alkylaminopropionates such as lauraminopropionic acid. Ammoniurn Sulfonate Amphoterics. This class of amphoteric emulsifiers refers to "sultaines" or "sulfobetaines", such as cocamidopropyl-hydroxysultaine.

Preferred emulsifiers are those that have an HLB (i.e., hydrophilic to lipophilic balance) of at least 4 and more preferably at least 6. Even more preferred emulsifiers are hydrophilic emulsifiers having an HLB in the range between 8 and 20, such as in the range between 10 and 20. Most preferred emulsifiers have an HLB of at least 12, such as at least 15. One or more emulsifiers may be used in the compositions of the present invention at a suitable level to produce the desired result. In a preferred embodiment, the one or more emulsifier are present in a total amount of at least 0.1 wt %, more preferably at least 0.5 wt %, and even more preferably at least 1.0 wt %, based on the total weight of the ready to use composition. In order to avoid irritation of a emulsifier in a preferred embodiment, the emulsifier is present in a total amount of no greater than 10 wt%, more preferably no greater than 5 wt%, even more preferably no greater than 3 wt%, and even more preferably no greater than 2 wt%, based on the total weight of the ready to use composition.

- Polymeric thickeners which may be added to the hydrophilic phase; e.g. gums such as acacia, alginates, carageenan, chitosan, collagen, tragacanth and xantham; celluloses, such as sodium carboxymethyl-, hydroxymethyl-, hydroxypropyl- and hydroxypropylmethyl celluloses; acrylic acids, such as carbomers and polycarbophil; colloidal solids such as silica, clays and microcrystalline cellulose; hydrogels such as polyvinyl alcohol and polyvinylpyrrolidone; thermoreversible polymers such as poloxamers.

- pH adjuster (buffering agents) which may be added to the hydrophilic phase, such as diethanolamine, lactic acid, monoethanolamine, triethanolamine, sodium hydroxide, sodium phosphate, citric acid, acetic acid, tartaric acid, hydrogen phosphoric acid, phosphate salts and diethylamine.

- Permeation enhancers, which may be added either to the hydrophilic or lipophilic phase in order to increase the penetration of Oxaprozin within stratum corneum.

- Preservatives, such as antimicrobial agents like benzalkoniumchloride, benzyl alcohol, chlorhexidine, imidazolidinyl urea, phenol, potassium sorbate, benzoic acid, bronopol, chlorocresol, parabens esters, phenoxyethanol and sorbic acid.

- Humectants, such as glycerol, glycerine, propylene glycol, sorbitol.

- Chelating agents, such as citric acid and edetic acid.

- Antioxidants, such as alfa-tocopherol, ascorbic acid, ascorbyl palmitate, butylated hydroxyanisole, butylated hydroxytoluene, sodium ascorbate, sodium metabisulphite

- Suspending agents that may be selected from the group comprising celluloses and cellulose derivatives such as, e.g., carboxymethyl cellulose, hydroxyethylcellulose, hydroxypropylcellulose, hydroxypropylmethylcellulose, carrageenan, acacia gum, arabic gum, tragacanth, and mixtures thereof.

- Gel-forming agents (Thickener). Suitable gel bases and viscosity-increasing components (thickeners) may be selected from the group comprising liquid paraffin, polyethylene, fatty oils, colloidal silica or aluminium, zinc soaps, glycerol, propylene glycol, tragacanth, carboxyvinyl polymers, magnesium-aluminium silicates, Carbopol®, hydrophilic polymers such as, e.g. starch, or cellulose derivatives such as, e.g., carboxymethylcellulose, hydroxyethylcellulose and other cellulose derivatives, water-swellable hydrocolloids, carrageenans, hyaluronates (e.g. hyaluronate gel optionally containing sodium chloride), and alginates including propylene glycol alginate. Still other examples are high molecular weight polysaccharide gum, such as xanthan gum.

In an embodiment of the invention, where the water-soluble salt of Oxaprozin constitutes a stability problem, for example due to phase-separation of the lipophilic and hydrophilic phase of an emulsion, it is important to select an emulsifier that is less sensitive to electrolytes. Therefore, in certain preferred embodiments, non-ionic emulsifiers are found to be useful in the compositions. Exemplary non-ionic emulsifiers include, but are not limited to polyol esters including glycols and glycerol esters; sorbitan derivatives including sorbitan esters of fatty acids and polyoxyethylene sorbitan esters; polyoxyethylene esters; polyoxyethylene ethers; poloxamers; nonylphenyl ethers. The preferred ones are sorbitan esters of fatty acids and polyoxyethylene sorbitan esters.

The physical stability can be recognised by observing the tendency of phase separation of the emulsion after challenging the emulsion to physical stress. For example, the emulsion can be exposed to repeating cycles of "freeze and thaw", for example 6 times, followed by centrifugation. Alternatively, the phase separation can be observed after prolonged storage of the emulsion at either 25°C, 40°C, or 60°C for 1 month, 3 months, 6 months, 12 months, optionally after centrifugation of the dermatological composition.

In a certain embodiment of the invention, the dermatological composition is an-oil-in-water emulsion, e.g. provided as a cream or liniment. The ratio between the hydrophilic and lipophilic phase is adapted in a manner so as to modify the diffusion/solubility of the water soluble salt within stratum corneum. Typically, the ratio of the hydrophilic phase to lipophilic phase ranges between 5:1 and 1:1 by weight, preferably between 4:1 and 1.4:1 by weight.

Typical examples on embodiments where the dermatological composition is provided as an emulsion, such as oil-in-water emulsion encompasses those comprising:

- from 0.5 % to 7.5%, preferably from 1 to 5% by weight of a salt of Oxaprozin or a closely related compound, wherein the salt is present in the hydrophilic phase; and a vehicle comprising one or more dermatologically acceptable ingredients;

- from 30% to 80%, preferably from 50 to 70% by weight of a hydrophilic phase comprising water, optionally a hydrophilic solvent (e.g. glycerol and/or propylene glycol) and a thickener (e.g. xanthan gum or Carbopol 2020™); and

- from 20 % to 70 %, preferably from 30% to 50% by weight of a hydrophobic phase comprising: a fatty component, an emulsifier or a mixture of emulsifiers, optionally one or more oily components, and optionally one or more lipophilic solvents,

with the proviso that all constituents make up 100% by weight of the composition.

In one certain embodiment of the invention, the dermatological composition is an emulsion, such as an oil-in-water emulsion, and comprising:

- from 0.5 % to 7.5%, preferably from 1 to 5% by weight of a salt of Oxaprozin or a closely related compound, wherein the salt is present in the hydrophilic phase; and a vehicle comprising one or more dermatologically acceptable ingredients;

- from 30% to 80%, preferably from 50 to 70% by weight of a hydrophilic phase comprising between 80 and 95% by weight of water, optionally a hydrophilic solvent (e.g. glycerol and/or propylene glycol) and a thickener (e.g. xanthan gum or Carbopol 2020™); and

- from 20 % to 70 %, preferably from 30% to 50% by weight of a hydrophobic phase comprising: a fatty component, an non-ionic emulsifier or a mixture of non-ionic emulsifiers, optionally one or more oily components, and optionally one or more lipophilic solvents,

with the proviso that all constituents make up 100% by weight of the composition.

More specifically, such compositions comprise:

- from 0.5 % to 7.5%, preferably from 1 to 5% by weight of a salt of Oxaprozin or a closely related compound, wherein the salt is present in the hydrophilic phase; and a vehicle comprising one or more dermatologically acceptable ingredients;

- from 30% to 80%, preferably from 50 to 70% by weight of a hydrophilic phase comprising between 80 and 95% by weight of water, a hydrophilic solvent (e.g. selected from propylene glycol, glycerol, sorbitol, ethanol, industrial methylated spirit, polyethylene glycols, propylene glycols, propylene carbonate and triacetin) and a thickener (e.g. selected from starch, cellulose derivatives (e.g. carboxymethylcellulose, hydroxyethylcellulose and other cellulose derivatives), water-swellable hydrocolloids, carrageenans, hyaluronates (e.g. hyaluronate gel optionally containing sodium chloride), alginates (including propylene glycol alginate) and high molecular weight polysaccharide gum (e.g. xanthan gum) and cross-linked polyacrylic acid copolymer (e.g. Carbopol 2020™); and

- from 20 % to 80 %, preferably from 30% to 50% by weight of a hydrophobic phase comprising: a fatty component (e.g. selected from animal fats, synthetic glycerides, waxes, lanolin, petrolatum, polyalkylsiloxanes and solid macrogols), an non-ionic emulsifier or a mixture of non-ionic emulsifiers (e.g. selected from Poloxamers, sorbitan esters of fatty acids, and polyoxyethylene sorbitan monostearate and mixtures thereof), optionally one or more oily components, and optionally one or more lipophilic solvents,

with the proviso that all constituents make up 100% by weight of the composition.

In a currently interesting embodiment of the invention, the dermatological composition is an emulsion, such as an oil-in-water emulsion that comprises:

- from 1 to 5% by weight of a salt of Oxaprozin or a closely related compound, wherein the salt is present in the hydrophilic phase; and a vehicle comprising one or more dermatologically acceptable ingredients;

- from 50 to 70% by weight of a hydrophilic phase comprising between 80 and 95% by weight of water, a hydrophilic solvent selected from glycerol and propylene glycol) and a thickener selected from high molecular weight polysaccharide gum (e.g. xanthan gum) and cross-linked polyacrylic acid copolymer (e.g. Carbopol 2020™); and

- from 20 % to 50 %, preferably from 30% to 50% by weight of a hydrophobic phase comprising: a fatty component (e.g. selected from animal fats, synthetic glycerides, waxes, lanolin, petrolatum, polyalkylsiloxanes and solid macrogols), an non-ionic emulsifier or a mixture of non-ionic emulsifiers (e.g. selected from Poloxamers, sorbitan esters of fatty acids, and polyoxyethylene sorbitan monostearate and mixtures thereof), optionally one or more oily components, and optionally one or more lipophilic solvents with the proviso that all constituents make up 100% by weight of the composition.

In another embodiment of the invention, the dermatological composition is essentially water free or contains less than 20% by weight of water, preferably less than 15, such as 10% or 5% by weight of water. Typically, such embodiments comprises from 0.5 % to 7.5%, preferably from 1 to 5% by weight of a salt of Oxaprozin or a closely related compound, and a vehicle comprising a solubiliser, such as transcutol and an oily component, optionally a fatty component, an oily component and a hydrophilic solvent.

Further examples of dermatological compositions are solutions, sprays, foams and aerosols that may be formulated according to a person skilled in pharmaceutical science.

Where it is desirable to administer the salt of the invention to rectum, the dermatological composition may be in the form of suppositories, crèmes, gels and enemas. Where it is desirable to administer the salt of the invention to vagina, the dermatological composition may be in the form of pessaries, moulded pessaries, vaginal capsules, vaginal tablets and the like.

Further usable dermatological compositions include shampoos, jellies, soaps, sticks, powders, films, pads, sponges, dressings, drenches, bandages and plasters which may be made according to the knowledge to a person skilled in pharmaceutical formulation science. Suitable powder components may be selected from the group comprising alginate, collagen, lactose, powder, which is able to form a gel when applied to a wound (absorbs liquid/wound exudate).

In preferred embodiments of the invention, the dermatological composition consists essentially of a water-soluble salt of Oxaprozin or a closely related compound and one or more dermatologically acceptable ingredient or carrier. As used herein, "consisting essentially of" means that the dermatological composition may include ingredients additional to the water soluble salt of Oxaprozin or a closely related compound, but only if the additional ingredients do not materially alter the basic and novel characteristics of the therapeutically effect of Oxaprozin or a closely related compound. Therefore, in some embodiments, Oxaprozin or a closely related compound is the sole therapeutically active ingredient of the dermatological composition.

It should be understood that in a preferred embodiment of the invention, the water-soluble salt is made with Oxaprozin in un-derivatized form. However, closely related compounds sharing the same basic molecular structure may be applied in managing dermatological diseases and to benefit from the water-soluble salt form. Therefore, the invention encompasses water-soluble salts of closely related compounds of Oxaprozin. Oxaprozin is chemically designated 4,5-diphenyl-2-oxazole-propionic acid, and has the following chemically structure:

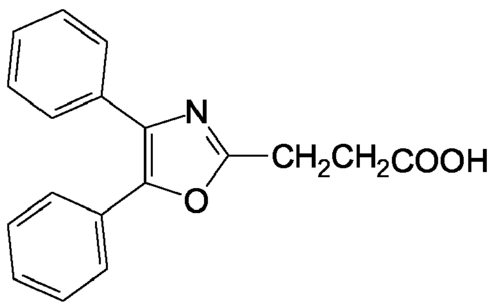

Several derivatives of Oxaprozin with anti-inflammatory effect have been described in the patent literature. General derivatives as well as the manufacturing thereof are described in US 3,578,671. Specific derivatives are described in US 5,380,738 (4-fluorophenyl and 4-methylsulfonylphenyl), US 4,659,728 (hydroxy substituted derivatives), US 6,090,834 (sulfonyl derivatives), US 3,506679 (4,5-diarylthiazol derivatives).

As used herein, the term "closely related compounds" includes compounds with the general formula I:

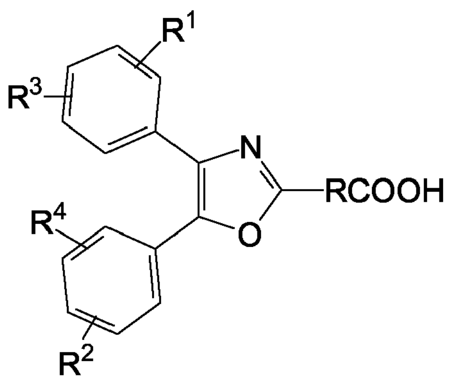

I

and bioisosters thereof.

Such compounds include various length of the R carbon chain implying that the propionic acid of the Oxaprozin molecule being replaced with acetic acid or butyric acid. Furthermore, the R chain and the two phenyl rings may be subject to substitution by replacing one or more hydrogen(s) with substituents defined herein. Finally, such compounds also include bioisosters where the oxygen of the oxazole ring is replaced with sulfur (S) to provide a thiazole ring. A bioisoster may also be provided by replacing the carboxylic acid group by a thioacid (COSH).

As used herein, the group R primarily defines straight-chained or branched, saturated or unsaturated aliphatic carbon chains containing 2 carbon atoms. Thus, n-propionic, iso-propionic and propionenic acids are anticipated. In closely related embodiments, the group R defines straight chained or branched, saturated or unsaturated aliphatic radicals containing 1 or 3 carbon atoms. Thus, acetic, acrylic and butyric acids are also anticipated.

Thus, R may be selected from $C_{1-3}$-alkyl, $C_{2-3}$-alkenyl, and $C_{2-3}$-alkynyl. The group R may be derivatized including substitution of one hydrogen atom with cyano (CN), halogen (Br, Cl, F, I), hydroxy (OH), amino ($NH_2$), and nitro ($NO_2$).

The terms "$R^1$, $R^2$, $R^3$ and $R^4$ are meant to define substituents of the phenyl rings of formula I so as to define un-substituted, mono-substituted or di-substituted phenyl rings, wherein $R^1$, $R^2$, $R^3$ and $R^4$ may be the same or different. The phrase "mono and di-substituted phenyl radicals" designates substitution of one or two hydrogen(s) in each phenyl ring with $R^1$ and/or $R^3$ in one ring and independently thereof with $R^2$ and/or $R^4$ in the second phenyl ring.

$R^1$ and $R^2$ independently designates radicals selected from hydrido, $C_{1-6}$-alkyl, $C_{2-6}$-alkenyl, $C_{2-6}$-alkynyl, $C_{1-6}$-alkoxyl, carboxy (CO), carboxyaldehyde (CHO), carboxy acid and a derivative thereof ($COR^5$), cyano (CN), halogen (Br, Cl, F, I), hydroxy (OH), hydroxy derivative (OR'), amino ($NH_2$), primary amino (NHR'), secondary amino (NR'R"), nitro ($NO_2$), sulfonyl ($HSO_2$) and sulfonyl derivative ($R^7$-$SO_2$), $R^7$ designates a substituent selected from $C_{1-6}$-alkyl, aryl, $NH_2$, NHR', NR'R".

$R^3$ and $R^4$ independently designates radicals selected from hydrido, $C_{1-6}$-alkyl, $C_{2-6}$-alkenyl, $C_{2-6}$-alkynyl, $C_{1-6}$-alkoxyl, CO, CHO, CO-Me, CO-Et, $COR^5$, CN, halogen, OH, OR', $NH_2$, NHR', NR'R" and $NO_2$. In a preferred embodiment, $R^3$ and $R^4$ independently designate radicals selected from hydrido, $C_{1-6}$-alkyl and $C_{2-6}$-alkenyl. In a still more preferred embodiment, $R^3$ and $R^4$ each designate hydrido.

As used herein, $R^5$ designates a group selected from hydroxy (OH), hydroxy derivative ($OR^6$) $NH_2$, NHR', NR'R", SH or $SR^6$.

$R^6$ designates a radical selected from $C_{1-6}$-alkyl, $C_{2-6}$-alkenyl and aryl.

R' and R" define the same or a different group selected from $C_{1-6}$-alkyl and $C_{2-6}$-alkenyl.

$R^7$ designates a substituent selected from $C_{1-6}$-alkyl, aryl, $NH_2$, NHR', NR'R".

The term "aryl" means phenyl, mono- or di-substituted phenyl wherein one or two hydrogens have been replaced by substituents selected from $C_{1-6}$-alkyl, $C_{2-6}$-alkenyl, $C_{2-6}$-alkynyl, $C_{1-6}$-alkoxyl, carboxy (CO), carboxyderivative (COR'), carboxyaldehyde (CHO), carboxylic acid (COOH), carboxylderivative (COOR') cyano (CN), halogen (Br, Cl, F, I), hydroxy (OH), amino ($NH_2$), primary amino (NHR'), secondary amino (NR'R") and nitro ($NO_2$). Preferably, the term "aryl" means phenyl or mono-substituted phenyl wherein one hydrogen have been replaced by substituents selected from $C_{1-6}$-alkyl, $C_{2-6}$-alkenyl, $C_{1-6}$-alkoxyl, CO, CHO, CN, halogen, OH, $NH_2$ and $NO_2$.

In the present context, $C_{1-3}$-alkyl is designated to define straight-chained or branched carbon chains having from 1 to 3 carbon atoms and wherein the carbon chain is situated between the oxazole ring and the $COOR^5$ group, such as $-CH_2-$, $-CH_2CH_2-$ and $-CH_2CH_2CH_2$ including isomers thereof. Likewise, $C_{2-3}$-alkenyl and $C_{2-3}$-alkynyl means unsaturated aliphatic carbon chain containing 2 or 3 carbon atoms, such as -CHCH-, -CC-, $-CHCHCH_2-$, $-CCCH_2-$, including isomers thereof.

$C_{1-6}$-alkyl is meant to define saturated, straight chained or branched alkyl radical containing the number of carbon atoms indicated, e.g. "1-6" means all alkyl radicals from methyl up to hexyl including all isomers thereof, e.g. iso-butenyl. Where applicable, the alkyl may be in cyclical form, such as cyclohexane.

$C_{2-6}$-alkenyl defines unsaturated straight-chained or branched alkylene radicals containing the number of carbon atoms indicated e.g. 1- or 2-propenyl, 1-, 2- or 3-butenyl and the like and isomers thereof.

$C_{2-3}$-alkynyl defines unsaturated chained or branched alkynyl radicals containing the number of carbon atoms indicated, e.g. ethynyl, 1- or 1-propynyl, 1-, 2- or 3-butynyl and the like and isomers thereof.

$C_{1-6}$-alkoxyl means alkoxy radicals containing up to 6 and preferably up to 4 carbon atoms, e.g. methoxy, ethoxy, propoxy etc.

The groups, $C_{1-6}$-alkyl, $C_{1-9}$-alkyl, $C_{2-6}$-alkenyl, $C_{2-9}$-alkenyl, $C_{2-6}$-alkynyl and $C_{2-9}$-alkynyl may optionally be mono-substituted with CN, CO, CHO, $COR^5$, halogen, OH, OR' $NH_2$, NHR', NR'R" and nitro, wherein $R^5$, $R^6$, R' and R" are as defined above.

The term halogen defines bromine, chlorine, fluorine and iodine.

The term "hydrido" designates a single hydrogen atom (H).

The compounds of the present invention may contain asymmetric carbon atoms, and, therefore, the instant invention may also include the individual diastereomers and enantiomers, which may be prepared or isolated by methods

known to those skilled in the art.

As mentioned, in preferred embodiments of the invention, the monoethanolamine salt is made with Oxaprozin.

In other interesting embodiments, Oxaprozin is provided as a derivative according to formula I. In one group of embodiments designated A, R is $-CH_2-$. In another group of embodiments designated B, R is selected from $C_2$-alkyl, $C_2$-alkenyl, and $C_2$-alkynyl, such as $-CH_2CH_2-$, -CHCH-, -CC-. In still another group of embodiments (designated C), R is selected from $C_3$-alkyl, $C_3$-alkenyl, and $C_3$-alkynyl, such as $-CH_2CH_2CH_2-$, $-CHCHCH_2-$, - $CCCH_2-$ and geometric and stereo isomers thereof. In all such embodiments (A, B and C), R may be substituted in that one hydrogen atom is replaced with CN, halogen, OH, $NH_2$, $NO_2$, preferably with OH. Furthermore, in such embodiments, $R^1$, $R^2$, $R^3$, $R^4$, $R^5$, $R^6$, $R^7$, R' and R" are as defined above.

In further interesting embodiments of A, B and C (designated AA, BA, CA), $R^2$ and $R^4$ independently designate radicals selected from hydrido, $C_{1-6}$-alkyl, halogen, OH and OR' and $R^1$, $R^3$, $R^5$, $R^6$, $R^7$, R' and R" are as defined above.

In other further interesting embodiments of A, B and C (designated AB, BB and CB), $R^2$ and $R^4$ is hydrido and $R^1$, $R^3$, $R^5$, $R^6$, $R^7$, R' and R" are as defined above.

In further interesting embodiments of A, AA, AB, B, BA, BB, C, CA and CB, the groups $R^1$, $R^2$, $R^3$, $R^4$, $R^5$, $R^6$, $R^7$, R' and R" are as defined under the respective groups of embodiments, but the term "aryl" is meant to denote phenyl or mono substituted phenyl, wherein one hydrogen has been replaced by substituents selected from $C_{1-6}$-alkyl, $C_{2-6}$-alkenyl, $C_{2-6}$-alkynyl, $C_{1-6}$-alkoxyl, CN, CO, CHO, COOH, halogen, OH, $NH_2$, NHR', NR'R" and $NO_2$.

In still further interesting embodiments of A, AA, AB, B, BA, BB, C, CA and CB, the groups $R^1$, $R^2$, $R^3$, $R^4$, $R^5$, $R^6$, $R^7$, R' and R" are as defined under the respective groups of embodiments, but the term "aryl" is meant to denote phenyl or mono substituted phenyl, wherein one hydrogen has been replaced by substituents selected from $C_{1-6}$-alkyl, $C_{1-6}$-alkoxyl, CN, CHO, COOH, halogen, OH, $NH_2$, and $NO_2$.

In still further interesting embodiments of A, AA, AB, B, BA, BB, C, CA and CB, the groups $R^2$ and $R^4$ independently designate radicals selected from hydrido, $C_{1-6}$-alkyl, $C_{1-6}$-alkoxyl, CN, COOH, halogen, OH, $NH_2$, NHR, NR'R", $NO_2$, $HSO_2$, $R^7$-$SO_2$ and $R^1$, $R^3$, $R^7$, R' and R" are as defined under the respective embodiments.

In still further interesting embodiments of A, AA, AB, B, BA, BB, C, CA and CB, the groups, $C_{1-6}$-alkyl, $C_{1-9}$-alkyl, $C_{2-6}$-alkenyl, $C_{2-9}$-alkenyl, $C_{2-6}$-alkynyl and $C_{2-9}$-alkynyl may optionally be mono-substituted with CN, halogen, OH, OR' $NH_2$, NHR', NR'R" and nitro and $R^1$, $R^2$, $R^3$, $R^4$, $R^5$, $R^6$, $R^7$ R' and R" are as defined under the respective embodiments. In such embodiments, the term "aryl" is meant to denote phenyl or mono substituted phenyl, wherein one hydrogen atom has been replaced by substituents selected from $C_{1-6}$-alkyl, $C_{1-6}$-alkoxyl, CN, CHO, COOH, halogen, OH, $NH_2$, and $NO_2$.

It should be understood that in still further interesting embodiments of the above all mentioned, R is $-CH_2-$ or $C_2$-alkyl or $C_2$-alkenyl.

The monoethanolamine salt of Oxaprozin or a closely related compound as defined above may be provided in the form of a pharmaceutically acceptable solvate, which may be a hydrate (comprising from half a mole of $H_2O$ up to about 10 moles of $H_2O$ per mole of salt) or comprise other solvents of crystallization such as alcohols.

Typical examples of Oxaprozin related compounds are:

4,5-diphenylthiazol-2-yl- propionic acid;
4,5-diphenyloxazol-2-yl-acrylic acid;
4,5-diphenyloxazol-2-yl-acetic acid;
4,5-di-(4'-chlorophenyl)-oxazol-2-yl-propionic acid;
4-(4'-bromophenyl)-5-phenyloxazole-2-yl-propionic acid;
4-(4-hydroxyphenyl-5-phenyl-2-oxazole propanoic acid;
4-(4-fluorophenyl)-5-[4-(methylsulfonyl)phenyl]-2-oxazolepropionic acid;
4-(4-fluorophenyl)-5-[4-(methylsulfonyl)-phenyl]-2-oxazoleacetic acid;
4-(4-fluorophenyl)-5-[4-(methylsulfonyl)phenyl]-2-oxazolebutanoic acid;
[4-(4-aminosulfonylphenyl)-5-(3,4-dichlorophenyl)]-2-oxazoleacetic acid;
[4-(4-aminosulfonylphenyl)-5-(3-chloro-4-fluorophenyl)]-2-oxazoleacetic acid;
[4-(4-aminosulfonylphenyl)-5-(3-fluoro-4-methoxyphenyl)]-2-oxazoleacetic acid;
[4-(4-aminosulfonylphenyl)-5-(4-chlorophenyl)]-2-oxazoleacetic acid;
[4-(4-aminosulfonylphenyl)-5-phenyl]-2-oxazolebutanoic acid;
[4-(4-aminosulfonylphenyl)-5-phenyl]-2-oxazolepropanoic acid;
[4-(4-aminosulfonylphenyl-5-(3,4-difluorophenyl)]-2-oxazoleacetic acid;
[4-(4-methylsulfonylphenyl)-5-phenyl]-2-oxazoleacetic acid;
[4-(4-methylsulfonylphenyl)-5-phenyl]-2-oxazolebutanoic acid;
[4-(4-methylsulfonylphenyl)-5-phenyl]-2-oxazolepropanoic acid;
4-[4-aminosulfonylphenyl)-5-(3-fluoro-4-methoxyphenyl)-2-oxazolyl] -alpha-bromoacetic acid;
5-(4-nitrophenyl-4-phenyl-2-oxazole-2-yl propionic acid;
5-(4'-fluorophenyl)-4-phenyloxazole-2-yl-propionic acid;

5-(4-hydroxyphenyl-4-phenyl-2-oxazole propanoic acid;
5-(4-fluorophenyl)-4-[4-(methylsulfonyl)phenyl]-2-oxazolepropionic acid;
[5-(4-aminosulfonylphenyl)-4-(4-chlorophenyl)]-2-oxazoleacetic acid;
[5-(4-aminosulfonylphenyl)-4-phenyl]-2-oxazoleacetic acid;
[5-(4-aminosulfonylphenyl)-4-phenyl]-2-oxazolebutanoic acid;
[5-(4-aminosulfonylphenyl)-4-phenyl]-2-oxazolepropanoic acid;
[5-(4-aminosulfonylphenyl)-4-phenyl]-2-oxazolepropionic acid.

In summary, the term "Oxaprozin or a closely related compound "is meant to include compounds according to formula I, which include metabolites of Oxaprozin, bioisosters thereof and derivatives thereof as defined above. Examples of metabolites of Oxaprozin are hydroxy substituted Oxaprozin, namely 5-(4-hydroxyphenyl)-4-phenyl-2-oxazolepropanoic acid, 4-(4-hydroxyphenyl)-5-phenyl-2-oxazolepropanoic acid and 4-(4-hydroxyphenyl)-5-(4-hydroxyphenyl)-2-oxazolepropanoic acid as described in US 4,659,728.

It should be understood that one interesting embodiment of the invention encompass dermatological compositions comprising i) a monoethanolamine salt of Oxaprozin or a closely related compound; and ii) a vehicle comprising one or more dermatologically acceptable excipients or carriers as mentioned above.

In accordance with the superior effect of the monoethanolamine salt, a further aspect of the invention relates to the monoethanolamine salt of Oxaprozin or a closely related compound.

The monoethanolamine Oxaprozin salt of the invention may be obtained by methods that are known per se.

One aspect of the invention relates to an advantageous method of preparing the salt of the invention comprising dissolving Oxaprozin and monoethanolamine in a solvent or mixture of solvents in which both are soluble, mixing the solutions of the individual compounds and subsequently precipitating the salt, optionally by removing the solvent by evaporation or optionally by cooling the solution. The yields obtained with this procedure are very high and the ease of handling theses makes the manufacturing price low.

Examples of suitable solvents for the manufacturing procedure of the invention include: alcohols such as lower alkanols, e.g. methanol or ethanol, ethers such as di-lower alkyl ethers, e.g. diethyl ether, cyclic ethers such as dioxane or tetrahydrofurane, ketones such as di-lower alkyl ketones, e.g. acetone, carboxylic acid esters such as lower alkanecarboxylic acid esters, e.g. ethyl acetate, amides such as N,N-di-lower alkylamides, e.g. N,N-dimethyl formamide, sulfoxides such as di-lower alkyl sulfoxides, e.g. dimethyl sulfoxide, or mixtures thereof or mixtures thereof with water.

*Uses and methods*

According to the present invention, the dermatological compositions of the invention can be used in the treatment of inflammatory dermatological diseases, preferably where at least one, preferably where at least two of the enzymes Protein tyrosine kinase Syk, Protein tyrosine kinase ZAP-70 and PDE-IV plays a role in mediating the inflammatory dermatological diseases. It is generally expected that such inflammatory dermatological diseases are caused by an initial hypersensitivity reaction in the skin, such as type-I allergy or type-IV allergy reactions in skin.

Accordingly, another aspect relates to the use of a dermatological composition of the invention for the preparation of a medicament intended to be topically applied to the skin for the treatment of an inflammatory dermatological disease, such as for the treatment of the inflammation of a dermatological disease, in particular for the treatment of eczemas.

According to the present invention, the inflammatory dermatological disease to be treated encompasses those where at least one of the enzymes Protein tyrosine kinase Syk, Protein tyrosine kinase ZAP-70 and PDE-IV plays a role in mediating the inflammatory dermatological diseases. It is generally expected that such inflammatory dermatological diseases are caused by an initial hypersensitivity reaction in the skin, such as type-I allergy or type-IV allergy reactions in skin.

Therefore, still another aspect of the invention relates to the use of a dermatological composition of the invention for the preparation of a medicament intended to be topically administered to the skin for the inhibition of one or more of the enzymes selected from Protein tyrosine kinase Syk; Protein tyrosine kinase ZAP-70 and/or PDE-IV in inflamed skin cells of a subject diagnosed with an inflammatory dermatological disease.

According to the underlying pharmacological principle of the invention, the administration of the dermatological composition to skin effectively alleviate, remove or prevent specific symptoms caused by the inflammation or the hypersensitivity reaction in a subject diagnosed or suffering from an inflammatory dermatological disease. For example, pruritus is a predominant and highly unpleasant symptom of many inflammatory diseases.

Therefore, an important aspect of the invention relates to the treatment or prevention of pruritus and optionally other significant symptoms associated with or caused by an inflammatory dermatological disease, such as particularly eczemas.

Accordingly, the invention relates to the use of a dermatological composition of the invention for preparation of a medicament intended to be topically administered to the skin for the alleviation, removal or prevention of one or

more of the symptoms in skin selected from pruritus; erythema; and/or scaling in a subject having an inflammatory dermatological disease, such as particularly a subject having eczema.

In the context of the present invention, the term "an inflammatory dermatological disease" is meant to define a dermatological disease, where at least one of the enzymes selected from the group consisting of Protein tyrosine kinase Syk; Protein tyrosine kinase ZAP-70 and PDE-IV enzyme play a role in mediating the dermatological disease.

The phrase "where at least one of the enzymes selected from the group consisting of Protein tyrosine kinase Syk; Protein tyrosine kinase ZAP-70 and PDE-IV enzyme play a role in mediating the dermatological disease" is meant to define various dermatological diseases where over-expression or excessive amounts of these enzymes play a role. The following dermatological diseases are meant as non-limiting examples of such dermatological diseases: acne vulgaris, adult eczema, alopecia, allergic contact dermatitis, allergic dermatitis, allergic contact eczema, asteatotic eczema, atopic eczema, hand eczema, atopic dermatitis, carcinomas, childhood eczema, chronic dermatitis of hands or feet, contact dermatitis, contact eczema, discoid eczema, insect bite inflammation, drug-induced skin reactions, dermatitis herpetiformis, discoid lupus erythematosus, eczema, epidermolysis bullosa, erythroderma, erythema nodosum, erythema multiforme, hand eczema, hand and foot dermatitis, ichthyosis vulgaris, infantile eczema, keratoconus, keratosis pilaris lichen simplex chronicus, lichen planus, nummular dermatitis, melanomas, over-treatment dermatitis, pemphigus, pemphigoid, photodermatoses, pityriasis rosea, pyoderma gangrenosum, pompholyx, psoriasis, prurigo nodularis, rosacea, scabies, seborrheic dermatitis, seborrhea, scleroderma, Sjogren's Disease, stasis dermatitis, subacute cutaneous lupus erythematosus, sunburn, cutaneous manifestations of systemic lupus erythematosus, vitiligo and urticaria.

The phrase "treating the inflammation of a dermatological disease" is meant to define that the treatment as described herein reduces, removes of prevents the inflammation associated with or caused by a dermatological disease. Characteristic signs of inflammation in a dermatological disease are oedema, erythema and scaling, which this invention reduces, removes or prevents.

The phrase "formulated for topical administration to skin" and topical administration is meant to define interchangeable terms that encompasses the formulation of the active ingredient of this invention (Oxaprozin or a closely related compound) into a dosage form that can be applied to skin of a subject and which result in the local presence of the active ingredient in the skin. The phrase "local presence of the active ingredient in skin" is meant to include topical administration of the active ingredient to skin with the presumption that systemic uptake of the active ingredient is limited or nil. Thus, it is intended that less than 25% by weight, such as less than 20% by weight, such as less than 15% by weight, such as less than 10%, 8%, 5% and 3% by weight, of the topically administered active ingredient enters the blood stream or is recovered in urine and faeces. Dosage forms for topical application to skin typically encompass emulsions (creams), ointments, gels, liniments, powders and solutions.

The term "skin cells" is meant to encompass cells present in stratum corneum, dermis and epidermis. When considering inflammatory dermatological diseases, such cells may include cells that constitute the inflammation in skin, such as T-cells, macrophages, mast cells, Langerhans cells and neutrophils.

The term "skin" is meant to include skin of the entire embody including the scalp, the forehead, the head, arms, legs, breast and so forth. The term "skin" is also meant to include various layers of the skin, such as stratum corneum, epidermis and dermis.

The term "subject" for purposes of treatment includes any subject, but is preferably a subject who is in need of the treatment of an inflammatory dermatological disease. For purposes of prevention, the subject is any subject, and preferably a subject that is at risk of, or is predisposed to, developing an inflammatory dermatological disease. The subject is typically an animal, and yet more typically is a mammal. "Mammal", as used herein, refers to any animal classified as a mammal, including humans, domestic and farm animals, zoo, sports, or pet animals, such as dogs, horses, cats, cattle, etc. Preferably, the mammal is a human. Typically, a subject is a human diagnosed or suffering from various forms of eczemas, such as contact dermatitis, atopic dermatitis and hand eczemas. In preferred embodiments the subject is a human, a dog, a cat or a horse.

The terms "treat", "treating" and "treatment" as used herein are meant to include alleviating or abrogating an inflammatory dermatological disease or its attendant symptoms and alleviating or eradicating the cause of the disease itself.

The terms "prevent", "preventing" and 'prevention", as used herein, refer to a method of delaying or precluding the onset of symptoms of an inflammatory dermatological disease, such as preventing the reoccurrence of inflammation or pruritus.

In presently interesting embodiments of the invention, the treatment or prevention of inflammatory dermatological diseases comprises inhibition of one or more of the enzymes Protein tyrosine kinase Syk; Protein tyrosine kinase ZAP-70 and/or PDE-IV in inflamed skin cells of said subject. As recognised by the present inventor, the treatment of an inflammatory dermatological disease is improved by inhibiting multiple targets rather than one target, for which reason an improved embodiment of the invention preferably relates to the treatment of dermatological diseases, where at least two of the above-mentioned enzymes play a role, such as the Protein tyrosine kinase Syk and the Protein tyrosine kinase ZAP-70, the Protein tyrosine kinase Syk and the PDE-IV enzyme; the Protein tyrosine kinase ZAP-70 and the PDE-IV

enzyme.

In order to provide a general treatment principle for dermatological diseases, such as eczemas, it may be advantageous to inhibit all of the above-mentioned enzymes.

Such interesting embodiments of the invention relates to the treatment of inflammatory dermatological diseases where hypersensitivity reactions are part of their pathology, such as particularly type-IV or type-I allergy reactions. Currently interesting examples of such dermatological diseases are eczemas and dermatitis, such as contact dermatitis, atopic dermatitis and hand eczema.

The meaning of the word "eczema" is "flowing over" which describes some of the above inflammatory changes. The word eczema and dermatitis are used somewhat interchangeable because in both conditions similar inflammatory conditions occur in the skin.

Eczema refers to several conditions that share a pattern of changes in the surface of the skin. Eczema appears as an episode of skin inflammation that may present itching, scaling and erythema of the skin. When it develops into a long-term condition (chronic eczema), it leads to thickening skin, scaling, flaking, dryness and colour changes. There are many types of eczema, depending on the cause, shape and location of the rash. Most are related to allergies or to contact with irritating chemicals. Some are associated with or caused by underlying medical conditions that cause fluid retention in the legs.

The term "dermatitis" is meant to define inflammation that affects the epidermis and the superficial dermis and is characterised by featuring red and hot skin. Some times, the dermatitis further features oedema producing intradermal vesicles; weeping of fluid on to the surface, crusting, scaling and fissuring.

Eczemas are divided into endogenous eczemas and exogenous eczemas. Endogenous eczemas encompasses at least atopic dermatitis including various forms thereof (infantile eczema, childhood eczema, adult eczema, keratosis pilaris, ichthyosis vulgaris), pompholyx, discoid eczema and nummular eczema. Exogenous eczemas encompass at least allergic contact dermatitis, irritant contact dermatitis, hand eczema. Additionally, the term "eczema" may also encompass stasis dermatitis, asteatotic eczema, lichen simplex chronicus, psoriasis, seborrheic dermatitis and seborrhea.

Thus, in presently interesting embodiments of the invention, the dermatological disease refers to various types of eczemas which at least encompass the following conditions: atopic dermatitis, hand eczema, infantile eczema, child hood eczema, adult eczema, keratosis pilaris, ichthyosis vulgaris, hand and foot dermatitis, keratoconus, pompholyx, discoid eczema, nummular eczema, allergic contact dermatitis, irritant contact dermatitis, over-treatment dermatitis, asteatotic eczema, stasis dermatitis, lichen simplex chronicus, prurigo nodularis, seborrheic dermatitis, seborrhea and psoriasis.

In more preferred embodiments of the invention, the dermatological disease refers to atopic dermatitis, hand eczema, infantile eczema, child hood eczema, adult eczema, keratosis pilaris, ichthyosis vulgaris, hand and foot dermatitis, keratoconus, pompholyx, discoid eczema, nummular eczema, allergic contact dermatitis, irritant contact dermatitis, over-treatment dermatitis and hand eczema.

According to the underlying pharmacological principle of this invention, methods and uses of the invention comprise inhibition of one or more of the enzymes selected from the group consisting of Protein tyrosine kinase Syk; Protein tyrosine kinase ZAP-70 and PDE-IV enzyme in inflamed skin cells of a subject diagnosed with or suffering from or in the risk of developing an inflammatory dermatological disease.

According to the invention, a dermatological composition can also be applied for the preparation of a medicament for the treatment of pruritus in skin. That is to say that the invention also embodies a method for treating pruritus in skin, comprising administering to skin of a subject in need thereof the dermatological composition of this invention.

The term "pruritus" is interchangeable with the term "itch" and defines a well known sensory state associated with the desire to scratch. The sensory state associated with pruritus is different from that of pain although pruritus and pain can be produced by a variety of chemical, mechanical, thermal or electrical stimuli. Itch and pain differ in that (1) itch, unlike pain, can only be evoked from the superficial layers of skin, mucosa, and conjunctiva, and (2) itch and pain usually do not occur simultaneously from the same skin region. For example, the application of histamine to skin produces itch but not pain. Furthermore, itch and pain are treated by different pharmacologically principles in that itch appears to be insensitive to opiate and non-steroidal anti-inflammatory drug (NSAID) treatment, both of which are effective in treating pain. Finally, itch occurs only in the skin; pain arises from deeper structures as well. Pruritus may be localised in various well defined areas of the skin, such as skin of the ankle, wrest, lips, hands, chest and the like, or it might be general pruritus not localised in a particular part of the skin.

Pruritus is often a predominant symptom of the above-mentioned eczemas and the present inventor has demonstrated complete relief of this symptom in subjects suffering from various types of eczemas mentioned herein, particularly contact dermatitis and atopic dermatitis. Furthermore, the pruritus associated with insect bite was completely alleviated following topical application of the dermatological composition.

Generally spoken pruritus may be associated with a plethora of dermatological diseases, irrespective of their nature or to what extent inflammation or hypersensitivity reactions are part of the pathology. Non-limiting examples on

such dermatological diseases are: acne vulgaris, adult eczema, alopecia, allergic contact dermatitis, allergic dermatitis, allergic contact eczema, asteatotic eczema, atopic eczema, hand eczema, atopic dermatitis, carcinomas, childhood eczema, chronic dermatitis of hands or feet, contact dermatitis, contact eczema, discoid eczema, insect bite inflammation, drug-induced skin reactions, dermatitis herpetiformis, discoid lupus erythematosus, eczema, epidermolysis bullosa, erythroderma, erythema nodosum, erythema multiforme, hand eczema, hand and foot dermatitis, ichthyosis vulgaris, infantile eczema, keratoconus, keratosis pilaris lichen simplex chronicus, lichen planus, nummular dermatitis, melanomas, over-treatment dermatitis, pemphigus, pemphigoid, photodermatoses, pityriasis rosea, pyoderma gangrenosum, pompholyx, psoriasis, prurigo nodularis, rosacea, scabies, seborrheic dermatitis, seborrhea, scleroderma, Sjogren's Disease, stasis dermatitis, subacute cutaneous lupus erythematosus, sunburn, cutaneous manifestations of systemic lupus erythematosus, vitiligo and urticaria.

Dermatological causes of pruritus often relates to hypersensitivity reactions in skin or allergic reactions, such a type-I or type-IV allergy reactions in skin. Thus, pruritus may be associated with atopic dermatitis and the various types thereof (atopic dermatitis, hand eczema, infantile eczema, childhood eczema, adult eczema, keratosis pilaris, ichthyosis vulgaris, hand and foot dermatitis, keratoconus, pompholyx, discoid eczema, nummular eczema) and allergic contact reactions, such as with contact dermatitis and the various types thereof (allergic contact dermatitis, irritant contact dermatitis and over-treatment dermatitis).

Typically, pruritus is an unpleasant symptom of insect bites and stings, bullous pemphigoid, cutaneous T-cell lymphoma, dermatitis herpetiformis, folliculitis, lichen planus, lichen simplex chronicus, pediculosis (lice infestation), prurigo nodularis, psoriasis, scabies, sunburn, urticaria and xerotic eczema.

Therefore, in one preferred embodiment of the invention, the treatment of pruritus in skin is associated with or caused by insect bites (such as insect bite inflammation), insect stings, bullous pemphigoid, cutaneous T-cell lymphoma, dermatitis herpetiformis, folliculitis, lichen planus, lichen simplex chronicus, pediculosis (lice infestation), prurigo nodularis, scabies, sunburn, and urticaria.

Still other embodiments refer to the treatment or prevention of eczemas and dermatitis which do not typically involve a hypersensitivity reaction, such as asteatotic eczema including senile pruritus, stasis dermatitis, psoriasis, seborrheic dermatitis and seborrhea.

In still further embodiments of the invention pruritus is a symptom of:

1) heat exposure, such as resulting in cholinergic urticaria (response to hot bath, fever, exercise) and miliaria rubra (prickly heat);

2) occupational exposure, such as caused by fibreglass, glyceryl monothioglycolate, methyl methacrylate (e.g., plexiglas), potassium dichromate in cements and dyes, rosins or epoxy resins in adhesives and rubber;

3) systemic medications such as with antifungal agents like fluconazole (Diflucan), itraconazole (Sporanox), ketoconazole (Nizoral), Aspirin, B vitamins, including niacinamide, drug hypersensitivity to rifampin (Rifadin), vancomycin (Vancocin), nitrates (food preservatives), quinidine and spinal narcotics (pruritus affecting face, neck, and upper chest);

4) water exposure, such as resulting in aquagenic pruritus (associated with polycythemia vera, itching within 15 minutes of any water contact), cholinergic urticaria (response to warm water), polycythemia vera, swimmer's itch (seven-day eruption after freshwater swimming).

Thus, in other relevant embodiments of the invention pruritus is associated with or caused by systemic medications and water exposure.

Furthermore, pruritus can be caused by an underlying systemic disease. A wide variety of systemic diseases can cause generalized pruritus without diagnostic skin lesions.

Therefore, the invention embodies the treatment of pruritus associated with or caused by a systemic diseases selected from diabetes, hyperthyroidism, hypothyroidism, disorders of the parathyroid gland, carcinoid syndrome, hepatic disease, pregnancy, intrahepatic cholestasis, obstructive jaundice (in biliary tract or extrahepatic), primary biliary cirrhosis, drug induced cholestasis, chronic renal failure, uraemia, polycythaemia vera, iron deficiency, Hodgkin's Disease, Mycosis fungoides, Lymphosarcoma, Chronic leukaemia, Myleomatosis, Paraproteinaemia, Mast cell disease, HIV, Sezary's syndrome (T-cell lymphoma), leukaemia, multiple myeloma, Waldenström's macroglobinaemia, mycosis fungoides, benign gammopathy, systemic mastocytosis, haematological and lymphoproliferative disorders,carcinomatosis, adenocarcinoma and squamous cell carcinoma of various organ, tumour of brain, multiple sclerosis and brain tumours.

Furthermore, the invention provides dermatological compositions further comprising a dermatological treatment agent to improve the effect of the Oxaprozin or a closely related compound.

Typical examples on dermatological treatment agents are antihistamines, anti-bacterial agents, anti-fungal

agents, anti-pruritus agents, anti-viral agents, agents for combating parasites, steroidal anti-inflammatory agents, non-steroidal anti inflammatory agents, anaesthetic agents, keratolytic agents, agents for combating free radicals, metal chelating agents, antidandruff agents, anti-acne vulgaris agents, substance P or bradykinin antagonists or NO-synthase inhibitors.

The invention is further described by the examples.

Example 1 describes a typical formulation of a dermatological composition of Oxaprozin in the form of its water-soluble salt (mono-ethanolamine salt) and the formation of the water-soluble salt of Oxaprozin.

Examples 2, 3 and 4 demonstrate the beneficial effect of topical application of Oxaprozin (as a water-soluble salt) in treating pruritus associated with contact dermatitis, atopic dermatitis, insect bite inflammation and psoriasis, respectively.

Example 5 demonstrates the new pharmacological properties of Oxaprozin, which could not be demonstrated for Bufexamac that only inhibits the PDE-IV enzyme.

Example 6 demonstrates the significant effect of Oxaprozin in preventing and treating experimental contact dermatitis in a dose related manner and with stronger effect than observed with Betamethasone 17-valerate.

Example 7 demonstrates that Oxaprozin is able to inhibition the formation of ear oedema in an experimental contact dermatitis model, but that no effect could be observed for Bufexamac.

Example 8 demonstrates that Oxaprozin are safe when applied topically to skin and do not cause sensitization reactions, photo toxicity or acute dermal irritation even when applied in high dose.

Example 9 demonstrates that an emulsion of Oxaprozin (Example 1) has good cutaneous tolerance even when applied consecutively in a concentration of 5% for 28 days.

Examples 10 and 11 refer to the clinical assessment of the effect of Oxaprozin in the treatment of hand eczema and contact dermatitis, respectively.

## EXAMPLES

### Example 1

The monoethanolamine salt of Oxaprozin was prepared according to the following advantageous method:

10.0 g Oxaprozin was dissolved in 230 ml ethyl acetate under mild heating.
2.3 g of monoethanolamine was dissolved in 30 ml ethyl acetate and added to the Oxaprozin solution under agitation. After a few seconds, a significant precipitation could be observed. The solution was allowed to cool for 60 minutes and the salt was collected by filtration and dried.

A topical pharmaceutical composition according to the invention was prepared by dissolving 2.5% or 5.0% of the monoethanolamine salt of Oxaprozin in the water phase of the topical emulsion with the following composition (w/w):

**Hydrophobic phase**

| | |
|---|---|
| Tween 80™ (Polyoxyethylene sorbitan monooleate) | 1% |
| Span 60™ (emulsifier of the sorbitan ester type) | 2% |
| Medium chain triglycerides (MCT) | 20% |
| Petrolatum, white | 10% |
| Paraffin, light | 10% |
| Cetanol | 4% |

**Hydrophilic phase**

| | |
|---|---|
| Oxaprozin monoethanolamine salt | 2.5% |
| Water | 42.5% |
| Xanthan gum | 0.5% |
| Glycerol | 2 % |
| Propylenglycol | 2% |
| Benzylalcohol | 0.5% |

The emulsion was prepared by first heating the lipophilic phase and some of the hydrophilic phase (xanthan gum and water) to 70 degrees Celsius, and mixing them. The remaining hydrophilic phase is heated to 50°C and added subsequently cooling them under agitation.

**Example 2**

A 71 year old male subject had been suffering from irritant contact dermatitis for more than 5 years. The dermatitis was usually situated on the legs. The symptoms of the dermatitis was erythema, scaling and significant pruritus. During the last 5 years the subject had regularly been treated with strong topical steroids with a relatively good therapeutic effect on the erythema, but with no short term effect on the pruritus. During an aggravation of the dermatitis associated with a strong itch, the subject initiated a treatment with the emulsion according to example 1 containing 5.0% of the monoethanolamine salt of Oxaprozin. The subject experienced an immediate and complete alleviation of the pruritus 20 minutes after application of the emulsion of example 1. To maintain this level of efficacy, the subject had to reapply the emulsion three times daily the first day and twice daily during the next two weeks, where the erythema and scaling were gradually reduced. After 14 days of treatment the erythema had completely gone and the treatment was stopped. Two weeks later the erythema had still not reappeared. This indicates a surprisingly good therapeutic effect not only on the pruritus, but also on the underlying disease.

A 3½ year old female had been suffering from atopic dermatitis for at least 2 years. The dermatitis was present in the face and on more than 30% of the body and was characterised by erythema and extensive pruritus. The subject had periodically been treated with hydrocortisone ointment or pimecrolimus cream with some effect on the erythema, but with no short term effect on the pruritus.

During an aggravation of the dermatitis with extensive pruritus, the subject was treated with the emulsion according to example 1 containing 2.5% of the monoethanolamine salt of Oxaprozin. 15 minutes after application of the emulsion, the subject experienced a complete alleviation of the pruritus, which lasted 8 hours. During the next week the treatment was repeated when needed, 1-3 times daily and every time a complete recovery from pruritus was observed. During the week of treatment a significant improvement of erythema was also observed indicating a surprisingly good therapeutic effect not only on the pruritus, but also on the underlying disease.

**Example 3**

A 37 year old female, which previously had experienced insect bite inflammation in skin with pruritus and oedema as predominant symptoms, was treated with the emulsion according to example 1 containing 2.5% of the monoethanolamine salt of Oxaprozin following an insect bite by a mosquito. This treatment completely alleviated the pruritus after 20 minutes of application of the emulsion and the oedema disappeared overnight.

Contrarily, treatment of previous mosquito attacks with hydrocortisone ointment did not satisfactorily reduce pruritus and oedema.

**Example 4**

A 32 year old male subject had been suffering from plaque psoriasis for more than two years. The disease was apparent on the elbows with erythema, scaling and significant pruritus. During an aggravation of the symptoms the subject initiated a week of twice daily treatment with the emulsion according to claim 1 containing 5.0% of the monoethanolamine salt of Oxaprozin. The subject experienced an immediate and significant relief of pruritus after the first treatment. This level of efficacy was maintained for the entire week of treatment. Furthermore a significant reduction of erythema and scaling was observed. Again this indicated a surprisingly good therapeutic effect not only on the pruritus, but also on the underlying disease.

**Example 5**

The anti-inflammatory potential of Oxaprozin was determined by evaluating the inhibitory activity of Oxaprozin against the enzymes *Phosphodiesterase PDEIV, Protein Tyrosine Kinase SYK and Protein Tyrosine kinase ZA70 (ZAP-70).* The enzyme assays were conducted by MSD Pharma Services.

The following concentration of Oxaprozin (as the monoethanolamine salt) resulted in 50% inhibition of the following enzymes ($IC_{50}$);

| Enzyme | MDS Pharma Service No: | $IC_{50}$ |
|---|---|---|
| Phosphodiesterase PDE IV | 154000 | 22 μM |
| Protein Tyrosine Kinase, SYK | 155761 | 28 μM |
| Protein Tyrosine Kinase, ZA70 (ZAP-70) | 155987 | 38 μM |

In comparison, Bufexamac only exhibits inhibitory effect on the PDE-IV enzyme.

**Example 6**

Screening for Anti-inflammatory Effect in the Oxazolone induced Mouse Ear Oedema Assay.

The anti-inflammatory activity of Oxaprozin was assessed by topical administration of Oxaprozin to oxazolone induced ear inflammation in mice. This screening method is commonly employed for screening and evaluation of anti-inflammatory drugs, in particular with respect to the inflammation seen in contact dermatitis. Betamethasone-17 valerate was used as the positive control.

Oxaprozin in the form of the monoethanolamine salt was administered topically as a dilution in acetone in a quantity of 250-1000μg/ear. Betamethasone was administered topically in quantities of 20μg/ear. Betamethasone was applied in the commercial form Celeston valerate ® 0.1%.

Test Procedure

Day 0

All groups were immunised with 20 μl oxazolone, 1.6% in ethanol 96% (w/v) on the left and the right ear.

Day 7

The ear thickness of all mice on both the left and right side was measured with an electronic measuring gauge. All groups were challenged with 20 μl oxazolone (1.6% in ethanol 96% (w/v)) on the left ear and the right ear. Vehicle (acetone) or test article solutions were administered 20 minutes before and 20 minutes after oxazolone challenge.

Day 8

24 hours after oxazolone challenge the ear thickness of all mice was measured with an electronic measuring gauge.

The groups, doses and animal numbers will be as follows:

| Group | Drug | Dose | Animal numbers |
|---|---|---|---|
| 1 | Vehicle, acetone | - | 1-10 |
| 2 | Monoethanolamine Oxaprozin | 1000μg/ear | 11-20 |
| 3 | Monoethanolamine Oxaprozin | 500μg/ear | 21-30 |
| 4 | Monoethanolamine Oxaprozin | 250μg/ear | 31-40 |
| 5 | Betamethasone 17-valerate | 20μg/ear | 41-50 |

Mean thickness of the ears and standard deviations were calculated. Ear swelling was calculated as the difference between the ear thickness day 7 and day 8. Percent inhibition of the ear swelling was assessed as the difference between the mean ear swelling of group 1 and the mean ear swelling of groups 2 to 5 expressed in percent.

Statistics

Differences in ear swelling between the vehicle treated group and the other groups were tested for significance employing a non-parametric statistical method of analysis, the Mann-Whitney U test. The required level of significance was $p<0.05$.

Results

The oxazolone challenge caused an inflammation in the ears, which was significant in the vehicle treated group after 24 hours since the ears were swollen and bright red. The test articles to some extent prevented the reaction. No adverse reactions to any of the test articles were observed.

Ear swelling

The various concentrations of the test articles inhibited the ear swelling as shown in the table below:

| Drug | Dose | % inhibition of ear swelling | Mann-Whitney U test |
|---|---|---|---|
| Vehicle, acetone | - | - | - |
| Monoethanolamine Oxaprozin | 1000μg/ear | 95 | P<0.001 |
| Monoethanolamine Oxaprozin | 500μg/ear | 77 | P<0.001 |
| Monoethanolamine Oxaprozin | 250μg/ear | 53 | P<0.001 |
| Betamethasone 17-valerate | 20μg/ear | 66 | P<0.001 |

Conclusion

The Oxaprozin monoethanolamine salt of the invention displayed a dose dependent and highly significant inhibition of ear swelling. The inhibition observed with the highest dose was significantly stronger than the inhibition obtained with Betamethasone 17-valerate in its clinically used dose level.
The data indicate that the Oxaprozin monoethanolamine salt of the invention has a strong suppressing effect on contact dermatitis.

**Example 7**

Comparison of Oxaprozin and Bufexamac in the Oxazolone induced Mouse Ear Oedema Assay.

The anti-inflammatory activity of Oxaprozin in comparison to Bufexamac was assessed using the same test method as described in Example 6. Both test articles were applied in a dose of 500 μg/ear and dissolved in 96% ethanol. Betamethasone, as the positive control was administered topically in quantities of 20μg/ear.

Results:

Oxaprozin showed a statistically significant inhibition of the formation of ear oedema, but Bufexamac did not inhibit the formation of ear oedema at all.

**Example 8**

**Acute dermal irritation**

A sample of Oxaprozin as the monoethanolamine salt was prepared in two concentrations (2.5% and 5% by weight) by dilution with water and tested for acute dermal irritation.

Procedure:

The sample was applied at a dose of 0.5 mL, on an undamaged skin area of the right flank of each animal. The patch was secured in position with a strip of surgical adhesive tape.

On the left flank an untreated area served as the control.
The skin reactions were evaluated after 1 hour and then after 24, 48 and 72 hours following removal of the patch according to the following grading scale:

Grading scales:

Erythema (0: No erythema, 1: Slight (barelyperceptible) erythema, 2: Definite erythema, 3: Moderate to severe erythema, 4: Severe erythema (purple) with formation of eschars (deep lesions) preventing erythema from being grading).
Oedema (0: No oedema, 1: Very slight (barely perceptible) oedema, 2: Slight oedema (contour clearly defined), 3: Moderate oedema (thickness), 4: Severe oedema (thickness greater than 1 mm, surface larger than zone of application)

Results:

With respect to Oxaprozin (2.5% by weight), no cutaneous reactions (erythema and oedema) were observed irrespective of the examination time.
With respect to Oxaprozin (5% by weight), only a slight erythema on the treated area at the reading time 1 hour was observed. This reaction was totally reversible between the 2nd and the 3rd day of the test.

**Photo toxicity:**

Test for photo toxicity is carried out to evaluate the risk of cutaneous reactions on the guinea pig following exposure to ultraviolet radiation.

Procedure:

Oxaprozin (supplied as its monoethanolamine salt) was diluted in water to produce solutions containing either 2.5% or 5% by weight of the Oxaprozin salt. The solution was applied at a dose of 0.5 mL over the whole right-hand flank of each guinea pig. Thirty minutes after the treatment, the animals were subjected to ultraviolet radiations (UV-B first and then UV-A).
The animals were irradiated with the irradiation source VLX 3W (Biotronic, Vilbert Lourmat) at the Maximal Non Erythemateous Dose (M.N.E.D) 7000 J/cm2 for UV-A and 150mJ/cm2for the UV-B.

Results:

A macroscopic evaluation of the cutaneous reactions (erythema and oedema) was conducted 24 and 48 hours after irradiation. No macroscopic cutaneous reaction was attributable to photo irritation as compared with the reactions noticed on the reference sites (8-Methoxypsoralen: positive reference and product alone: negative reference). Thus, Oxaprozin is not phototoxic.

**Skin Sensitization**

Test for skin sensitization is carried out according to the Magnusson and Kligman method (J. Invest. Dermatol. 1969. 52, 268-276) and in accordance with O.E.C.D. Guideline N° 406 of July 17th, 1992, and the test method B.6 of the 96/54 E.E.C Directive.

Procedure:

Oxaprozin (supplied as its monoethanolamine salt) was diluted in water to produce solutions containing 2.5% by weight of the Oxaprozin salt.
Albino guinea pigs of Dunkin-Hartley strain were exposed to the test item after an acclimatisation period of at least five days.
The Maximum Non Necrotizing Concentration (M.N.N.C.) was determined by injecting by intradermal route the following concentrations: 2.5%, 1.25%, 0.625%, 0.3125%, 0.1562% and 0.078% diluted in physiological saline solution.
Pre-Maximum Non Irritant Concentration (pre-M.N.I.C.) was determined by application of the test item under an occlusive dressing during 24 hours, at the following concentrations: 2.5%, 1.25%, 0.625%, 0.3125% diluted in physiological saline solution.
Maximum Non Irritant Concentration (M.N.I.C.) was determined by initially establishing an induction phase by intradermal injection with a physiological saline solution and by topical application of distilled water followed by a 18-day rest phase. In the challenge phase where the test item is under occlusive dressing for 24 hours, the test item was applied to the skin of the Albino guinea pigs at the following concentrations: 2.5%, 1.25%, 0.625%, 0.3125% diluted in physiological saline solution.

Results:

No macroscopic cutaneous reactions attributable to allergy was recorded during the examination following the removal of the occlusive dressing (challenge phase) from the animals of the treated group. No cutaneous intolerance reaction was recorded in animals from the negative control group. Thus, Oxaprozin monoethanolamine salt is found to not causing sensitization reactions.

**Example 9**

Cutaneous tolerance of an emulsion (Example 1) containing Oxaprozin (as the monoethanolamine salt) 2.5% and 5% by weight, respectively, was tested by daily application at a dose of 2 ml per animal per day for 28 consecutive days on undamaged skin of rabbits.

Macroscopic cutaneous examinations were performed daily during the 28 days just before the daily application of the emulsion. Skin erythema, oedema, dryness, elasticity and skin fold thickness were assessed.

The results obtained showed slight erythema and oedema after some days of treatment but was totally reversed before the 19th and 10th day, respectively. Dryness was noted too in the beginning of the treatment and there was also observed slight skin fold thickening. The investigator concluded that the emulsion, both in 2.5% and 5% concentration, presented good dermal cutaneous tolerance after repeated application for 28 days.

**Example 10**

The efficacy of Oxaprozin or a related compound to treat and prevent hand eczema can be tested in a blinded treatment with the study preparation or vehicle twice daily for 4 weeks in 2 treatment groups with chronic hand dermatitis. Half of the patients will be treated with a cream formulation of Oxaprozin, e.g. Oxaprozin monoethanolamine salt in a concentration of 2.5% and the second half with the cream vehicle. Clinical assessment will be performed on day 1 prior to the first treatment (baseline) and following 1, 2, 3 and 4 weeks of treatment. Additionally the patients will answer a questionnaire for determination of the Dermatology Life Quality Index, a patient global assessment will be performed. The dosage to be applied is approximately 25 mg per day and the total dosage amounts to approximately 700 mg.

The clinical assessment can be done according to the HECSI scoring system that is an objective and accurate assessment of the severity of hand eczema. It incorporates both the extent and the intensity of the disease. Each hand is divided into five areas (fingertips, fingers (except the tips), palms, back of hand and wrists). For each of these areas the intensity of each of the clinical signs erythema, induration/papulation, vesicles, fissuring, scaling and oedema is graded on the following four point scale:

0 = no skin changes, 1 = mild disease, 2 = moderate, 3 = severe

For each location (total of both hands) a score from 0 to 4 is given for the extent of clinical symptoms with respect to the percentual affected area:

0 = 0 %, 1 = 1 – 25 %, 2 = 26 – 50 %, 3 = 51 – 75 %, 4 = 76 – 100 %

Finally the score given for the extent at each location is multiplied by the sum of the intensity of each clinical feature (Erythema (E), Infiltration/papulation (I), Vesicles (V), Fissures (F), Scaling (S), Oedema (O).

**Example 11**

The efficacy of Oxaprozin or a related compound to treat and prevent contact dermatitis can be assessed clinically in humans in a randomized, controlled double-blind study using test persons with known nickel allergy and with healthy skin in the test area. According to a standard procedure, at least three test fields of healthy skin located on the back are assigned to each test person, in which fields' allergy is provocated by application of nickel II sulfate vaseline

and test medication is applied in order to test efficacy. The test medication can be a cream formulation of Oxaprozin, e.g. Oxaprozin monoethanolamine salt in a concentration of 2.5% or 5% where the daily dose applied to the test field is approximately 15 mg and 40 mg, respectively. As positive control can be used Betamethasone 17- valerate Cream 0.1%, where the daily dose applied is approximately 0.6 mg Betamethasone/day. Furthermore, active ingredient-free vehicle is also tested. On day 1, study medication, positive control and vehicle is applied to test fields (= pretreatment) in that approximately 200 μl of each study preparation will be applied to the respective test fields, for example by using special test chambers (Finn Chambers®, Epitest Ltd. Oy, Finland, 18 mm inside in diameter.
On the consecutive day the pre-treated test fields will be treated with two concentrations of nickel II sulfate vaseline to induce an allergic reaction or with vaseline for 1 hours. The treatment with the different concentrations of nickel II sulfate vaseline will be performed in an smaller area in the middle of the defined pre-treated test fields using smaller test chambers, e.g. Finn Chambers®, Epitest Ltd. Oy, Finland, 12 mm inside in diameter. Approximately 30 μl nickel II sulfate vaseline or vaseline will be used. After this induction the efficacy of preventative treatment with the study preparations will be assessed. On study day 4 to 7 the test fields will be treated as described for day 1 with the study preparations once daily to assess the efficacy in the treatment of contact dermatitis. The extent of epidermal barrier impairment measured by TEWL, skin redness measured by chromametry and clinical skin condition evaluated by scoring will be determined. In addition photo documentation will be performed. Clinical assessment will be performed according to the following score:
0 = no reaction, 1 = erythema, but no induration, 2 = erythema, induration, discrete papules possible, 3 = erythema, induration, papules, vesicle, 4 = erythema, induration, confluencing vesicle.

Transepidermal water loss (evaporimetry) is a widely used non-invasive method for evaluation of skin impairment. The epidermis of healthy intact skin represents a barrier that minimizes external water loss. Any impairment of this barrier results in an increase of permeability to water with a corresponding increase of TEWL. Increased TEWL values are to be expected in the lesional test fields in subjects with allergic reactions induced by the treatment with nickel II sulfate vaseline.

## Claims

1. A dermatological composition comprising:

Oxaprozin or a closely related compound provided in a form of a water-soluble salt, wherein the closely related compound is defined by the general formula I:

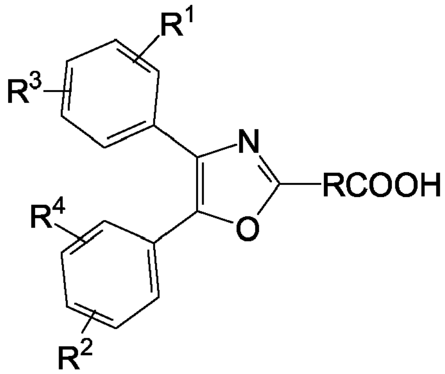

I

and
ii) a vehicle comprising one or more dermatologically acceptable ingredient(s) or carrier(s), wherein
R is selected from $C_{1-3}$-alkyl, $C_{2-3}$-alkenyl, and $C_{2-3}$-alkynyl and R is optionally derivatized by substitution of one hydrogen atom with CN, halogen OH, $NH_2$ and $NO_2$;
$R^1$ and $R^2$ independently designate radicals selected from hydrido, $C_{1-6}$-alkyl, $C_{2-6}$-alkenyl, $C_{2-6}$-alkynyl, $C_{1-6}$-alkoxyl, CO, CHO, CO-Me, CO-Et, CN, halogen, OH, OR', $NH_2$, NHR', NR'R", $NO_2$, $HSO_2$ and $R^7$ -$SO_2$;
$R^3$ and $R^4$ independently designate radicals selected from hydrido, $C_{1-6}$-alkyl and $C_{2-6}$-alkenyl;
$R^5$ designate radicals selected from OH, $OR^6$, $NH_2$, NHR', NR'R", SH and $SR^6$;
$R^6$ designate radicals selected from $C_{1-6}$-alkyl, $C_{2-6}$-alkenyl and aryl;

$R^7$ designate radicals selected from $C_{1-6}$-alkyl, aryl, $NH_2$, NHR' and NR'R";
R' and R" designate the same or different group selected from $C_{1-6}$-alkyl and $C_{2-6}$-alkenyl; and
"aryl" means phenyl or mono-substituted phenyl wherein one hydrogen have been replaced by substituents selected from $C_{1-6}$-alkyl, $C_{2-6}$-alkenyl, $C_{1-6}$-alkoxyl, CO, CHO, CN, halogen, OH, $NH_2$ and $NO_2$;
and wherein the oxygen of the oxazole ring optionally is replaced with sulfur (S) to provide a thiazole ring.

**2.** The composition according to claim 1, wherein the salt is a base addition salt selected from $Li^+$, $Na^+$, $K^+$, $Ca^{++}$, $Mg^{++}$, $Cu^+$, $Zn^+$, $Mn^+$, alkylphenylamine, ammonia, 2-aminoethanol, aminopyrimidine, aminopyridine, arginine, benethamine, benzathine, betaine, caffeine, choline, deanol, diethanolamine, diethylamine, 2-diethylaminoethanol, 2-dimethylaminoethanol, ethylenediamine, N- ethylmorpholine, N-ethylpiperidine, glucamine, glucosamine, glycinol, hydrabamine, imidazol, isopropylamine, meglumine, methylglucamine, morpholine, piperazine, piperidine, procaine, purine, pyrrolidine, theobromine, thiamine, triethanolamine, triethylamine, trimethylamine, tripropylamine, tromethamine, spermidine, glycine, alanine, valine, leucine, isoleucine, norleucine, tyrosine, cystine, cysteine, methionine, proline, hydroxy proline, histidine, omithine, lysine, arginine, serine, threonine, phenylalanine and guanidine salts.

**3.** The composition according to claim 1, wherein the salt is a base addition salt formed wherein the base is an organic amine having a pKa in the range between 8 and 12.

**4.** The composition according to claim 1, wherein the salt is the 2-aminoethanol salt of Oxaprozin or a closely related compound.

**5.** The composition according to any of the preceding claims, wherein the water-soluble salt of Oxaprozin or a closely related compound is in an amount ranging between 0.5% and 10% by weight of the composition.

**6.** The composition according to any one of the claims 1 to 4, wherein the water-soluble salt of Oxaprozin or a closely related compound is in an amount of about 2.5% by weight.

**7.** The composition according to any one of the claims 1 to 4, wherein the water-soluble salt of Oxaprozin or a closely related compound is in an amount of about 5% by weight.

**8.** The composition according to any one of the preceding claims, wherein the pH ranges between 6.5 and 7.8.

**9.** The composition according to any one of the claims 1 to 7, wherein the pH of the hydrophilic phase ranges between 6.5 and 7.8.

**10.** The composition according to any one of the preceding claims, wherein the vehicle further comprising at least 30 % by weight of a hydrophobic phase comprising a fatty component and/or an oily component.

**11.** The composition according to claim 10, wherein the fatty component is selected from the group consisting of beeswax, paraffin, petrolatum, triglycerides, sorbitan esters of fatty acids, solid macrogols and condensation products between sorbitan esters of fatty acids and ethylene oxide.

**12.** The composition according to claim 10, wherein the oily component is selected from the group consisting of almond oil, castor oil, cacao butter, coconut oil, corn oil, cottonseed oil, linseed oil, olive oil, palm oil, peanut oil, poppy seed oil, rapeseed oil, sesame oil, soybean oil, sunflower oil, and teaseed oil), mineral oils, fatty oils, liquid paraffin, mineral oil, isopropyl myristate, beewax, cottonseed oil, cetosteraryl alcohol, lanolin, white soft paraffin, yellow soft paraffin, canola oil, cetyl alcohol (cetanol), peanut oil, oleic acid, isopropyl palmitate, castor oil, stearyl alcohol, jojoba oil, stearic acid and silicone oils.

**13.** The composition according to any one of the preceding claims formulated as an emulsion, a gel, a solution, a liniment, an ointment, a spray, an aerosol or a powder.

**14.** The composition according to any one of the claims 1 to 12, wherein the vehicle is an emulsion.

**15.** The composition according to claim 14, wherein the emulsion comprises a non-ionic emulsifier.

**16.** The composition according to claim 15, wherein the non-ionic emulsifier is selected from the group consisting of polyol esters including glycols and glycerol esters; sorbitan derivatives including sorbitan esters of fatty acids and

polyoxyethylene sorbitan esters; polyoxyethylene esters; polyoxyethylene ethers; poloxamers; nonylphenyl ethers.

**17.** The composition according to claim 15, wherein the non-ionic emulsifier is selected from the group consisting of sorbitan esters of fatty acids and polyoxyethylene sorbitan esters.

**18.** The composition according to claim 15, wherein the vehicle comprises from 30% to 80% by weight of a hydrophilic phase comprising between 80 and 95% by weight of water, a thickener and optionally a hydrophilic solvent; and from 20 % to 70 % by weight of a hydrophobic phase comprising a fatty component, an non-ionic emulsifier or a mixture of non-ionic emulsifiers, optionally one or more oily components, and optionally one or more lipophilic solvents.

**19.** The composition according to any one of preceding claims, wherein the closely related compound is selected from the group consisting of
4,5-diphenylthiazol-2-yl- propionic acid;
4,5-diphenyloxazol-2-yl-acrylic acid;
4,5-diphenyloxazol-2-yl-acetic acid;
4,5-di-(4'-chlorophenyl)-oxazol-2-yl-propionic acid;
4-(4'-bromophenyl)-5-phenyloxazole-2-yl-propionic acid;
4-(4-hydroxyphenyl-5-phenyl-2-oxazole propanoic acid;
4-(4-fluorophenyl)-5-[4-(methylsulfonyl)phenyl]-2-oxazolepropionic acid;
4-(4-fluorophenyl)-5-[4-(methylsulfonyl)-phenyl]-2-oxazoleacetic acid;
4-(4-fluorophenyl)-5-[4-(methylsulfonyl)phenyl]-2-oxazolebutanoic acid;
[4-(4-aminosulfonylphenyl)-5-(3,4-dichlorophenyl)]-2-oxazoleacetic acid;
[4-(4-aminosulfonylphenyl)-5-(3-chloro-4-fluorophenyl)]-2-oxazoleacetic acid;
[4-(4-aminosulfonylphenyl)-5-(3-fluoro-4-methoxyphenyl)]-2-oxazoleacetic acid;
[4-(4-aminosulfonylphenyl)-5-(4-chlorophenyl)]-2-oxazoleacetic acid;
[4-(4-aminosulfonylphenyl)-5-phenyl]-2-oxazolebutanoic acid;
[4-(4-aminosulfonylphenyl)-5-phenyl]-2-oxazolepropanoic acid;
[4-(4-aminosulfonylphenyl-5-(3,4-difluorophenyl)]-2-oxazoleacetic acid;
[4-(4-methylsulfonylphenyl)-5-phenyl]-2-oxazoleacetic acid;
[4-(4-methylsulfonylphenyl)-5-phenyl]-2-oxazolebutanoic acid;
[4-(4-methylsulfonylphenyl)-5-phenyl]-2-oxazolepropanoic acid;
4-[4-aminosulfonylphenyl)-5-(3-fluoro-4-methoxyphenyl)-2-oxazolyl] -alpha-bromoacetic acid;
5-(4-nitrophenyl-4-phenyl-2-oxazole-2-yl propionic acid;
5-(4'-fluorophenyl)-4-phenyloxazole-2-yl-propionic acid;
5-(4-hydroxyphenyl-4-phenyl-2-oxazole propanoic acid;
5-(4-fluorophenyl)-4-[4-(methylsulfonyl)phenyl]-2-oxazolepropionic acid;
[5-(4-aminosulfonylphenyl)-4-(4-chlorophenyl)]-2-oxazoleacetic acid;
[5-(4-aminosulfonylphenyl)-4-phenyl]-2-oxazoleacetic acid;
[5-(4-aminosulfonylphenyl)-4-phenyl]-2-oxazolebutanoic acid;
[5-(4-aminosulfonylphenyl)-4-phenyl]-2-oxazolepropanoic acid;
[5-(4-aminosulfonylphenyl)-4-phenyl]-2-oxazolepropionic acid; and a pharmaceutically acceptable salt thereof.

**20.** A salt between 2-aminoethanol and Oxaprozin or a derivative thereof, wherein the derivative thereof is defined by the general formula I in claim 1 and wherein R $R^1$, $R^2$, $R^3$, $R^4$, $R^5$, $R^6$, $R^7$, R', R", "aryl" and bioisoster are as defined in claim 1.

**21.** Use of a dermatological composition as defined in any one of claims 1 to 19 for the preparation of a medicament for the treatment of an inflammatory dermatological disease in a subject.

**22.** The use according to claim 21, wherein the inflammatory dermatological disease is associated with or caused by a hypersensitivity reaction in the skin.

**23.** The use according to claim 21, wherein the inflammatory dermatological disease is associated with or caused by a type-I allergy or type-IV allergy reaction in the skin.

**24.** The use according to claim 21, wherein the inflammatory dermatological disease is selected from the group consisting of acne vulgaris, adult eczema, alopecia, allergic contact dermatitis, allergic dermatitis, allergic contact eczema,

asteatotic eczema, atopic eczema, hand eczema, atopic dermatitis, carcinomas, childhood eczema, chronic dermatitis of hands or feet, contact dermatitis, contact eczema, discoid eczema, insect bite inflammation, drug-induced skin reactions, dermatitis herpetiformis, discoid lupus erythematosus, eczema, epidermolysis bullosa, erythroderma, erythema nodosum, erythema multiforme, hand eczema, hand and foot dermatitis, ichthyosis vulgaris, infantile eczema, keratoconus, keratosis pilaris lichen simplex chronicus, lichen planus, nummular dermatitis, melanomas, over-treatment dermatitis, pemphigus, pemphigoid, photodermatoses, pityriasis rosea, pyoderma gangrenosum, pompholyx, psoriasis, prurigo nodularis, rosacea, scabies, seborrheic dermatitis, seborrhea, scleroderma, Sjogren's Disease, stasis dermatitis, subacute cutaneous lupus erythematosus, sunburn, cutaneous manifestations of systemic lupus erythematosus, vitiligo and urticaria.

**25.** The use according to claim 21, wherein the inflammatory dermatological disease is eczema.

**26.** The use according to claim 25, wherein eczema is selected from the group consisting of atopic dermatitis, hand eczema, infantile eczema, child hood eczema, adult eczema, keratosis pilaris, ichthyosis vulgaris, hand and foot dermatitis, keratoconus, pompholyx, discoid eczema, nummular eczema, allergic contact dermatitis, irritant contact dermatitis, over treatment dermatitis, hand eczema, asteatotic eczema, stasis dermatitis, lichen simplex chronicus, seborrheic dermatitis, seborrhea and psoriasis.

**27.** Use of the dermatological composition as defined in any one of claims 1 to 19 for the preparation of a medicament for the treatment of pruritus in skin of a subject.

**28.** The use according to claim 27, wherein pruritus is associated with or caused by a dermatological disease.

**29.** The use according to claim 27, wherein pruritus is associated with or caused by a hypersensitivity reaction in skin.

**30.** The use according to claim 27, wherein pruritus is associated with or caused by type-IV or type-I allergy reaction in skin.

**31.** The use according to claim 27, wherein pruritus is associated with or caused by a dermatological disease selected from the group consisting of acne vulgaris, adult eczema, alopecia, allergic contact dermatitis, allergic dermatitis, allergic contact eczema, asteatotic eczema, atopic eczema, hand eczema, atopic dermatitis, carcinomas, childhood eczema, chronic dermatitis of hands or feet, contact dermatitis, contact eczema, discoid eczema, insect bite inflammation, drug-induced skin reactions, dermatitis herpetiformis, discoid lupus erythematosus, eczema, epidermolysis bullosa, erythroderma, erythema nodosum, erythema multiforme, hand eczema, hand and foot dermatitis, ichthyosis vulgaris, infantile eczema, keratoconus, keratosis pilaris lichen simplex chronicus, lichen planus, nummular dermatitis, melanomas, over-treatment dermatitis, pemphigus, pemphigoid, photodermatoses, pityriasis rosea, pyoderma gangrenosum, pompholyx, psoriasis, prurigo nodularis, rosacea, scabies, seborrheic dermatitis, seborrhea, scleroderma, Sjogren's Disease, stasis dermatitis, subacute cutaneous lupus erythematosus, sunburn, cutaneous manifestations of systemic lupus erythematosus, vitiligo and urticaria.

**32.** The use according to claim 27, wherein pruritus is associated with or caused by eczema.

**33.** The use according to claim 32, wherein eczema is selected from the group consisting of atopic dermatitis, hand eczema, infantile eczema, child hood eczema, adult eczema, keratosis pilaris, ichthyosis vulgaris, hand and foot dermatitis, keratoconus, pompholyx, discoid eczema, nummular eczema, allergic contact dermatitis, irritant contact dermatitis and over treatment dermatitis.

**34.** The use according to claim 27, wherein pruritus is associated with or caused by asteatotic eczema, senile pruritus, stasis dermatitis, psoriasis, seborrheic dermatitis and seborrhea.

**35.** The use according to claim 27, wherein pruritus is associated with or caused by a dermatological diseases selected from the group consisting of insect bite inflammation, bullous pemphigoid, cutaneous T-cell lymphoma, dermatitis herpetiformis, folliculitis, lichen planus, lichen simplex chronicus, pediculosis, prurigo nodularis, scabies, sunburn and urticaria.

**36.** The use according to claim 27, wherein pruritus is associated with or caused by systemic medications.

**37.** The use according to claim 27, wherein pruritus is associated with or caused by exposure to water.

**38.** The use according to claim 27, wherein pruritus is associated with or caused by a systemic disease selected from the group consisting of diabetes, hyperthyroidism, hypothyroidism, disorders of the parathyroid gland, carcinoid syndrome, hepatic disease, pregnancy, intrahepatic cholestasis, obstructive jaundice (in biliary tract or extrahepatic), primary biliary cirrhosis, drug induced cholestasis, chronic renal failure, uraemia, polycythaemia vera, iron deficiency, Hodgkin's Disease, Mycosis fungoides, Lymphosarcoma, Chronic leukaemia, Myleomatosis, Paraproteinaemia, Mast cell disease, HIV, Sezary's syndrome (T-cell lymphoma), leukaemia, multiple myeloma, Waldenström's macroglobinaemia, mycosis fungoides, benign gammopathy, systemic mastocytosis, haematological and lymphoproliferative disorders,carcinomatosis, adenocarcinoma and squamous cell carcinoma of various organ, tumour of brain, multiple sclerosis and brain tumours.

**39.** The use according to any of claims 21 to 38, wherein the subject is a human, a dog, a cat or a horse.

European Patent Office

# EUROPEAN SEARCH REPORT

Application Number
EP 06 11 2007

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | US 2003/082226 A1 (SAMOUR CARLOS M ET AL) 1 May 2003 (2003-05-01)<br>* abstract *<br>* page 3, paragraph 69 - page 4, paragraph 71 * | 1-39 | INV.<br>A61K31/421<br>A61K31/426<br>A61P17/00<br>A61P17/02<br>A61P17/04<br>A61P17/06<br>A61P17/08<br>A61P17/10 |
| X | US 6 030 643 A (ADAMS ET AL) 29 February 2000 (2000-02-29)<br>* abstract *<br>* claim 1 * | 1-20 | |
| X,D | US 4 465 838 A (MUGHAL ET AL) 14 August 1984 (1984-08-14)<br>* abstract *<br>* claims 1,2 *<br>* example 3 * | 1-20 | |
| X | EP 0 270 316 A (PFIZER INC) 8 June 1988 (1988-06-08)<br>* claims 1,2,8; examples 3,5 * | 1-39 | TECHNICAL FIELDS SEARCHED (IPC) |
| X,D | WO 02/074290 A (AGIS INDUSTRIES LTD; ARKIN, MOSHE; ZIGHELBOIM, MARCEL; LAVON, ILANA;) 26 September 2002 (2002-09-26)<br>* abstract; claims 1,3 * | 1-39 | A61K |
| X | BROWN K ET AL: "Diaryloxazole and diarylthiazolealkanoic acids: two novel series of non-steroidal anti-inflammatory agents."<br>NATURE. 13 JUL 1968,<br>vol. 219, no. 150,<br>13 July 1968 (1968-07-13), page 164, XP009065622<br>ISSN: 0028-0836<br>* figure II; table 1 * | 1-20 | |
| | -/-- | | |

The present search report has been drawn up for all claims

| Place of search | Date of completion of the search | Examiner |
|---|---|---|
| Munich | 15 May 2006 | Nyeki, A |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

6

European Patent Office

# EUROPEAN SEARCH REPORT

Application Number
EP 06 11 2007

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | GREEN A Y ET AL: "Wy 23205, a new non-steroidal anti-inflammatory agent." BRITISH JOURNAL OF PHARMACOLOGY. AUG 1971, vol. 42, no. 4, August 1971 (1971-08), pages 638P-639P, XP009065608 ISSN: 0007-1188 * page 639, lines 1-4 * ----- | 1-20 | |
| X,D | US 3 578 671 A (KEVAN BROWN) 11 May 1971 (1971-05-11) * abstract * * examples 1-5,8-11 * * column 6, lines 60-63,69-72 * * column 7, lines 1-9,12,13 * * claims 1-12 * ----- | 1-20 | |
| X,D | US 5 380 738 A (NORMAN ET AL) 10 January 1995 (1995-01-10) * abstract * * claims 1,6,18,19 * ----- | 1-20 | TECHNICAL FIELDS SEARCHED (IPC) |
| X,D | US 4 659 728 A (LEWIS ET AL) 21 April 1987 (1987-04-21) * abstract * * claim 3 * ----- | 1-20 | |
| X,D | US 6 090 834 A (TALLEY ET AL) 18 July 2000 (2000-07-18) * abstract * * column 2; figure I * * column 3, lines 32-36 * * column 6, lines 19-28,31,32 * * column 12, lines 29,30,35,36,41,42 * * column 18, lines 3,4,13,14,36,37,40,41,44,45 * * examples 16-19,36-41,43,66 * ----- -/-- | 1-39 | |

The present search report has been drawn up for all claims

| Place of search | Date of completion of the search | Examiner |
|---|---|---|
| Munich | 15 May 2006 | Nyeki, A |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons
& : member of the same patent family, corresponding document

6

EPO FORM 1503 03.82 (P04C01)

European Patent Office

# EUROPEAN SEARCH REPORT

Application Number
EP 06 11 2007

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X,D | US 3 506 679 A (JOHN F. CAVALLA ET AL) 14 April 1970 (1970-04-14) * abstract * * example 2 * * claims 1,3 * ----- | 1-20 | |
| | | | TECHNICAL FIELDS SEARCHED (IPC) |

The present search report has been drawn up for all claims

| Place of search | Date of completion of the search | Examiner |
|---|---|---|
| Munich | 15 May 2006 | Nyeki, A |

6

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons
& : member of the same patent family, corresponding document

EPO FORM 1503 03.82 (P04C01)

## ANNEX TO THE EUROPEAN SEARCH REPORT ON EUROPEAN PATENT APPLICATION NO. EP 06 11 2007

This annex lists the patent family members relating to the patent documents cited in the above-mentioned European search report.
The members are as contained in the European Patent Office EDP file on
The European Patent Office is in no way liable for these particulars which are merely given for the purpose of information.

15-05-2006

| Patent document cited in search report | | Publication date | Patent family member(s) | | | Publication date |
|---|---|---|---|---|---|---|
| US 2003082226 | A1 | 01-05-2003 | NONE | | | |
| US 6030643 | A | 29-02-2000 | NONE | | | |
| US 4465838 | A | 14-08-1984 | AU | 544975 | B2 | 27-06-1985 |
| | | | AU | 8237882 | A | 04-11-1982 |
| | | | CA | 1169867 | A1 | 26-06-1984 |
| | | | CS | 236674 | B2 | 15-05-1985 |
| | | | DD | 202017 | A5 | 24-08-1983 |
| | | | DE | 3264400 | D1 | 01-08-1985 |
| | | | DK | 183582 | A | 29-10-1982 |
| | | | EP | 0063884 | A1 | 03-11-1982 |
| | | | ES | 8307230 | A1 | 16-10-1983 |
| | | | FI | 821324 | A | 29-10-1982 |
| | | | GR | 75913 | A1 | 02-08-1984 |
| | | | HU | 185276 | B | 28-12-1984 |
| | | | IE | 52909 | B1 | 13-04-1988 |
| | | | IL | 65443 | A | 30-04-1985 |
| | | | JP | 1634863 | C | 20-01-1992 |
| | | | JP | 2061466 | B | 20-12-1990 |
| | | | JP | 57181072 | A | 08-11-1982 |
| | | | KR | 8800871 | B1 | 28-05-1988 |
| | | | MY | 59987 | A | 31-12-1987 |
| | | | NZ | 200247 | A | 19-10-1984 |
| | | | PH | 18053 | A | 18-03-1985 |
| | | | PL | 236145 | A1 | 06-12-1982 |
| | | | PT | 74794 | A | 01-05-1982 |
| | | | US | 4532253 | A | 30-07-1985 |
| | | | ZA | 8202355 | A | 30-11-1983 |
| | | | ZW | 7482 | A1 | 09-11-1983 |
| EP 0270316 | A | 08-06-1988 | AU | 583754 | B2 | 04-05-1989 |
| | | | AU | 8206187 | A | 09-06-1988 |
| | | | DK | 634787 | A | 05-06-1988 |
| | | | IE | 873289 | L | 04-06-1988 |
| | | | JP | 63145238 | A | 17-06-1988 |
| | | | NO | 875045 | A | 06-06-1988 |
| | | | NZ | 222797 | A | 26-10-1990 |
| | | | PT | 86253 | A | 01-01-1988 |
| | | | ZA | 8709082 | A | 26-07-1989 |
| WO 02074290 | A | 26-09-2002 | CA | 2440687 | A1 | 26-09-2002 |
| | | | EP | 1414429 | A2 | 06-05-2004 |
| | | | US | 2003039704 | A1 | 27-02-2003 |
| US 3578671 | A | 11-05-1971 | NONE | | | |

EPO FORM P0459

For more details about this annex : see Official Journal of the European Patent Office, No. 12/82

## ANNEX TO THE EUROPEAN SEARCH REPORT ON EUROPEAN PATENT APPLICATION NO.

EP 06 11 2007

This annex lists the patent family members relating to the patent documents cited in the above-mentioned European search report. The members are as contained in the European Patent Office EDP file on
The European Patent Office is in no way liable for these particulars which are merely given for the purpose of information.

15-05-2006

| Patent document cited in search report | | Publication date | Patent family member(s) | | | Publication date |
|---|---|---|---|---|---|---|
| US 5380738 | A | 10-01-1995 | AT | 221054 | T | 15-08-2002 |
| | | | AU | 6949594 | A | 20-12-1994 |
| | | | CA | 2161769 | A1 | 08-12-1994 |
| | | | DE | 69431056 | D1 | 29-08-2002 |
| | | | DE | 69431056 | T2 | 16-01-2003 |
| | | | DK | 699192 | T3 | 04-11-2002 |
| | | | EP | 0699192 | A1 | 06-03-1996 |
| | | | ES | 2180580 | T3 | 16-02-2003 |
| | | | JP | 8510736 | T | 12-11-1996 |
| | | | PT | 699192 | T | 31-12-2002 |
| | | | WO | 9427980 | A1 | 08-12-1994 |
| | | | US | 5719163 | A | 17-02-1998 |
| US 4659728 | A | 21-04-1987 | NONE | | | |
| US 6090834 | A | 18-07-2000 | NONE | | | |
| US 3506679 | A | 14-04-1970 | IE | 32084 | B1 | 04-04-1973 |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- US 2005014729 A1
- WO 05009342 A2
- WO 05027977 A2
- JP 7316075 A
- WO 0059475 A1
- WO 02074290 A
- US 2005232957 A1
- WO 9531968 A
- WO 0205815 A
- WO 9709973 A
- US 5804572 A
- WO 0062743 A
- WO 0069255 A
- WO 04056349 A
- WO 0069406 A
- WO 02069963 A
- WO 02096435 A
- WO 03059347 A
- WO 03061704 A
- WO 03105806 A
- EP 1424325 A
- EP 1426059 A
- WO 04056305 A
- WO 04093796 A
- WO 9810742 A
- US 20030157138 A
- WO 9744022 A
- US 4465838 A
- US 3578671 A
- US 53807384 B
- US 4659728 A
- US 6090834 A
- US 3506679 A


### Non-patent literature cited in the description


- **GREAVES MW.** Pharmacology and significance of nonsteroidal anti-inflammatory drugs in the treatment of skin diseases. *J Am Acad Dermatol,* April 1987, vol. 16 ((4)), 751-64
- **DALLEGRI ; FRANCO.** A review of the emerging profile of the anti-inflammatory drug oxaprozin. *Expert Opin Pharmacother,* May 2005, vol. 6 ((5)), 777-85
- **GOING SM ; GUYER BM ; JARVIE DR ; HUNTER JA.** Salicylic acid gel for scalp psoriasis. *Clin Exp Dermatol,* May 1986, vol. 11 ((3)), 260-2
- **THOMSEN JS ; SIMONSEN L ; BENFELDT E ; JENSEN SB ; SERUP J.** The effect of topically applied salicylic compounds on serotonin-induced scratching behaviour in hairless rats. *Exp Dermatol,* August 2002, vol. 11 ((4)), 370-5
- **LOWE NJ ; VIRGADAMO F ; STOUGHTON RB.** Anti-inflammatory properties of a prostaglandin antagonist, a corticosteroid and indomethacin in experimental contact dermatitis. *BrJ Dermatol,* April 1977, vol. 96 ((4)), 433-8
- **BLACKAK ; HENSBY CN ; GREAVES MW.** The effect of topical flurbiprofen on human skin inflammation [proceedings]. *Br J Clin Pharmacol,* January 1980, vol. 9 (1), 125P
- **HUMPHREYS F ; SPIRO J.** The effects of topical indomethacin and clobetasol propionate on post-cryotherapy inflammation. *BrJ Dermatol,* May 1995, vol. 132 ((5)), 762-5
- **GREEN CA ; SHUSTER S.** Lack of effect of topical indomethacin on psoriasis. *Br J Clin Pharmacol,* September 1987, vol. 24 ((3)), 381-4
- **GEBHARDT M ; WOLLINA U.** Cutaneous side-effects of nonsteroidal anti-inflammatory drugs (NSAID). *Z Rheumatol,* November 1995, vol. 54 ((6)), 405-12
- **RIVERS JK ; MCLEAN DI.** An open study to assess the efficacy and safety of topical 3% diclofenac in a 2.5% hyaluronic acid gel for the treatment of actinic keratoses. *Arch Dermatol,* October 1997, vol. 133 ((10)), 1239-42
- **KAWAI S.** Cyclooxygenase selectivity and the risk of gastrointestinal complications of various non-steroidal anti-inflammatory drugs. A clinical consideration. *In Inflamma. Res.,* 1998, vol. 2, S102-S106
- **MAGNUSSON ; KLIGMAN.** *J. Invest. Dermatol.,* 1969, vol. 52, 268-276